# EUROPEAN PATENT SPECIFICATION

(11) **EP 3 750 331 B1**
(45) Date of publication and mention of the grant of the patent: **23.04.2025**
(21) Application number: 19750809.6
(22) Date of filing: 06.02.2019
(51) Int. Cl.: A61N 1/36, A61B 5/12, H04R 25/00, A61B 5/00

(54) **PROSTHETIC COGNITIVE ABILITY INCREASER**
VORRICHTUNG ZUR STEIGERUNG DER KOGNITIVEN FÄHIGKEIT MITTELS EINER PROTHESE
DISPOSITIF D'AUGMENTATION DE LA CAPACITÉ COGNITIVE AU MOYEN D'UNE PROTHÈSE

(30) Priority: 06.02.2018 US 201862626958 P
(43) Date of publication of application: 16.12.2020
(73) Proprietor: Cochlear Limited, Macquarie University, New South Wales 2109 (AU)
(72) Inventor: VON BRASCH, Alexander, Macquarie University, New South Wales 2109 (AU); FUNG, Stephen, Macquarie University, New South Wales 2109 (AU)
(74) Representative: Grünecker Patent- und Rechtsanwälte PartG mbB
(86) International application number: PCT/IB2019/050945
(87) International publication number: WO 2019/155374

(56) References cited:
- US-A1- 2011 022 119
- US-A1- 2014 286 513
- US-A1- 2016 100 796
- US-A1- 2016 119 726
- US-A1- 2017 056 655
- US-A1- 2017 056 655
- US-A1- 2017 180 895
- US-A1- 2017 347 209

## Description

### CROSS-REFERENCE TO RELATED APPLICATIONS

This application claims priority to U.S. Provisional Application No. 62/626,958, entitled PROSTHETIC COGNITIVE ABILITY INCREASER, filed on February 6, 2018, naming Alexander VON BRASCH of Macquarie University, Australia as an inventor.

### BACKGROUND

Hearing loss, which may be due to many different causes, is generally of two types: conductive and sensorineural. Sensorineural hearing loss is due to the absence or destruction of the hair cells in the cochlea that transduce sound signals into nerve impulses. Various hearing prostheses are commercially available to provide individuals suffering from sensorineural hearing loss with the ability to perceive sound. One example is a cochlear implant.

Conductive hearing loss occurs when the normal mechanical pathways that provide sound to hair cells in the cochlea are impeded, for example, by damage to the ossicular chain or the ear canal. Individuals suffering from conductive hearing loss may retain some form of residual hearing because the hair cells in the cochlea may remain undamaged.

Individuals suffering from hearing loss typically receive an acoustic hearing aid. Conventional hearing aids rely on principles of air conduction to transmit acoustic signals to the cochlea. In particular, a hearing aid typically uses an arrangement positioned in the recipient's ear canal or on the outer ear to amplify a sound received by the outer ear of the recipient. This amplified sound reaches the cochlea causing motion of the perilymph and stimulation of the auditory nerve. Cases of conductive hearing loss typically are treated by means of bone conduction hearing aids. In contrast to conventional hearing aids, these devices use a mechanical actuator that is coupled to the skull bone to apply the amplified sound.

In contrast to hearing aids, which rely primarily on the principles of air conduction, certain types of hearing prostheses commonly referred to as cochlear implants convert a received sound into electrical stimulation. The electrical stimulation is applied to the cochlea, which results in the perception of the received sound.

Many devices, such as medical devices that interface with a recipient, have structural and/or functional features where there is utilitarian value in adjusting such features for an individual recipient. The process by which a device that interfaces with or otherwise is used by the recipient is tailored or customized or otherwise adjusted for the specific needs or specific wants or specific characteristics of the recipient is commonly referred to as fitting. Optimization of stimulation parameters in electrically-stimulating auditory prostheses is disclosed in US 2017/056655 A1. Further examples for fitting of cochlear implants are disclosed in US 2016/100796 A1. US2016/119726 A1 shows controlling a hearing assistance device using a brain computer interface.

### SUMMARY

The invention is defined by the claims.

Methods disclosed hereinafter are not part of the present invention, but are useful for understanding the present invention.

In accordance with an exemplary embodiment, there is a method, comprising obtaining respective first reactions of a recipient to a series of sounds subjected to the recipient of a hearing prosthesis, the first reactions being directly related to the recipient's ability to hear the series of sounds; obtaining respective second reactions of the recipient to the series of sounds, the second reactions being different in kind than the first reactions; and fitting the hearing prosthesis based at least in part on both the first reactions and the second reactions.

In accordance with another exemplary embodiment, there is a method, comprising evoking first hearing percepts during a first temporal period utilizing a hearing prosthesis, wherein the hearing prosthesis operates based on a first set of operating parameters when evoking the first hearing percepts, receiving input indicative of an average cognitive load of the recipient resulting from the evoking of the first hearing percepts, and evoking second hearing percepts during a second temporal period utilizing the hearing prosthesis, wherein the hearing prosthesis operates based on a second set of operating parameters when evoking the second hearing percepts, wherein a switch from the first set of operating parameters to the second set of operating parameters is executed to increase the average cognitive load on the recipient that results from the evoking of the second hearing percepts.

In accordance with another exemplary embodiment, there is a method, including evoking first hearing percepts during a first temporal period utilizing a hearing prosthesis, wherein the first temporal period is a period in which the recipient effectively habilitates or rehabilitates his/her hearing with the hearing prosthesis, and wherein operating parameters of the hearing prosthesis are adjusted during the first temporal period to maintain, on average, a heightened cognitive load in the recipient of the hearing prosthesis resulting from use of the hearing prosthesis.

In accordance with another exemplary embodiment, there is a system, comprising a hearing prosthesis suite, and data input suite configured to receive data indicative of a cognitive load of the recipient, wherein the system is configured to operate in a hearing rehabilitative exercise machine mode in which the system automatically adjust operation of the hearing prosthesis based on data obtained by the data input suite to exercise the recipient, thereby rehabilitating the recipient.

### BRIEF DESCRIPTION OF THE DRAWINGS

Embodiments are described below with reference to the attached drawings, in which:
FIG. 1 is a perspective view of an exemplary hearing prosthesis in which at least some of the teachings detailed herein are applicable;
FIG. 2 presents an exemplary flowchart according to an exemplary embodiment;
FIG. 3 presents an exemplary system according to an exemplary embodiment;
FIGs. 4, 5, and 6 present various devices and systems according to an exemplary embodiment;
FIGs. 7-10 present exemplary algorithms for exemplary methods according to exemplary embodiments;
FIGs. 11 and 12 present exemplary data according to some exemplary scenarios of implementing the teachings detailed herein; and
FIGs. 13-18 present additional exemplary algorithms for exemplary methods according to exemplary embodiments.

### DETAILED DESCRIPTION

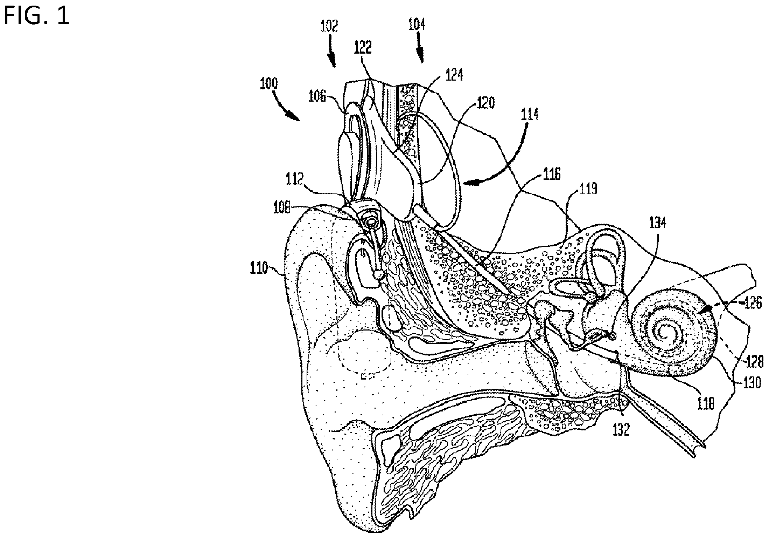

FIG. 1 is a perspective view of a cochlear implant, referred to as cochlear implant system 100, implanted in a recipient, to which some embodiments detailed herein and/or variations thereof are applicable. The cochlear implant system can include external components in some embodiments, such as external component 102, and implanted component (internal/implantable component) 104, as will be detailed below. Additionally, it is noted that the teachings detailed herein are also applicable to other types of hearing prostheses, such as by way of example only and not by way of limitation, bone conduction devices (percutaneous, active transcutaneous and/or passive transcutaneous), direct acoustic cochlear stimulators, middle ear implants, and conventional hearing aids, etc. It is noted that the teachings detailed herein are also applicable to so-called multi-mode devices. In an exemplary embodiment, these multi-mode devices apply both electrical stimulation and acoustic stimulation to the recipient. In an exemplary embodiment, these multi-mode devices evoke a hearing percept via electrical hearing and bone conduction hearing. Accordingly, any disclosure herein with regard to one of these types of hearing prostheses corresponds to a disclosure of another of these types of hearing prostheses or any medical device for that matter, unless otherwise specified, or unless the disclosure thereof is incompatible with a given device based on the current state of technology. Thus, the teachings detailed herein are applicable, in at least some embodiments, to partially implantable and/or totally implantable medical devices that provide a wide range of therapeutic benefits to recipients, patients, or other users, including hearing implants having an implanted microphone, auditory brain stimulators, pacemakers, visual prostheses (e.g., bionic eyes), sensors, drug delivery systems, defibrillators, functional electrical stimulation devices, catheters, etc.

In view of the above, it is to be understood that at least some embodiments detailed herein and/or variations thereof are directed towards a body-worn sensory supplement medical device (e.g., the hearing prosthesis of FIG. 1, which supplements the hearing sense, even in instances when there are no natural hearing capabilities, for example, due to degeneration of previous natural hearing capability or to the lack of any natural hearing capability, for example, from birth). It is noted that at least some exemplary embodiments of some sensory supplement medical devices are directed towards devices such as conventional hearing aids, which supplement the hearing sense in instances where some natural hearing capabilities have been retained, and visual prostheses (both those that are applicable to recipients having some natural vision capabilities and to recipients having no natural vision capabilities). Accordingly, the teachings detailed herein are applicable to any type of sensory supplement medical device to which the teachings detailed herein are enabled for use therein in a utilitarian manner. In this regard, the phrase sensory supplement medical device refers to any device that functions to provide sensation to a recipient irrespective of whether the applicable natural sense is only partially impaired or completely impaired, or indeed never existed.

Cochlear implant system 100 comprises an external component 102 and an internal/implantable component 104. In this example, the implantable component 104 is a cochlear implant.

The external component 102 is directly or indirectly attached to the body of the recipient and typically comprises an external coil 106 and, generally, a magnet (not shown in FIG. 1) fixed relative to the external coil 106. The external component 102 also comprises one or more sound input elements 108 (e.g., microphones, telecoils, etc.) for detecting sound signals or input audio signals, and a sound processing unit 112. The sound processing unit 112 includes, for example, a power source (not shown in FIG. 1) and a sound processor (also not shown in FIG. 1). The sound processor is configured to process electrical signals generated by a sound input element 108 that is positioned, in the depicted embodiment, by auricle 110 of the recipient. The sound processor provides the processed signals to coil 106 via, for example, a cable (not shown in FIG. 1).

The cochlear implant 104 comprises an implant body 114, a lead region 116, and an elongate intra-cochlear stimulating assembly 118. The implant body 114 comprises a stimulator unit 120, an internal/implantable coil 122, and an internal receiver/transceiver unit 124, sometimes referred to herein as transceiver unit 124. The transceiver unit 124 is connected to the implantable coil 122 and, generally, a magnet (not shown) fixed relative to the internal coil 122.

The magnets in the external component 102 and cochlear implant 104 facilitate the operational alignment of the external coil 106 with the implantable coil 122. The operational alignment of the coils enables the implantable coil 122 to transmit/receive power and data to/from the external coil 106. More specifically, in certain examples, external coil 106 transmits electrical signals (e.g., power and stimulation data) to implantable coil 122 via a radio frequency (RF) link. Implantable coil 122 is typically a wire antenna coil comprised of multiple turns of electrically insulated single-strand or multi-strand platinum or gold wire. The electrical insulation of implantable coil 122 is provided by a flexible molding (e.g., silicone molding). In use, transceiver unit 124 may be positioned in a recess of the temporal bone of the recipient. Various other types of energy transfer, such as infrared (IR), electromagnetic, capacitive and inductive transfer, may be used to transfer the power and/or data from an external device to a cochlear implant and, as such, FIG. 1 illustrates only one example arrangement.

Elongate stimulating assembly 118 is configured to be at least partially implanted in cochlea 130 and includes a plurality of longitudinally spaced intra-cochlear electrical stimulating contacts (electrical contacts) 128 that collectively form a contact array 126. Stimulating assembly 118 extends through an opening in the cochlea 130 (e.g., cochleostomy 132, the round window 134, etc.) and has a proximal end connected to stimulator unit 120 via lead region 116 that extends through mastoid bone 119. Lead region 116 couples the stimulating assembly 118 to implant body 114 and, more particularly, stimulator unit 120.

In general, the sound processor in sound processing unit 112 is configured to execute sound processing and coding to convert a detected sound into a coded signal that represents the detected sound signals. Since these encoded data are used by the cochlear implant 104 to generate stimulation signals, and because these signals vary dynamically according to the sound signals, the encoded data signals generated by the sound processor are sometimes referred to herein as "processed audio signals."

The processed audio signals generated by the sound processor are provided to the stimulator unit 120 via the RF link between the external coil 106 and the internal coil 122. The stimulator unit 120 includes one or more circuits that converts the processed audio signals, received via the transceiver unit 124, into sets of electrical stimulation signals (current stimulation) that are delivered via one or more stimulation channels that terminate in the stimulating contacts 128 (i.e., the sets stimulation signals are delivered to the recipient via the stimulating contacts 128). In this way, cochlear implant system 100 stimulates the recipient's auditory nerve cells, bypassing absent or defective hair cells that normally transduce acoustic vibrations into neural activity. Each set of stimulation signals represents a "stimulation cycle" that provides audio information to the recipient.

The stimulator unit 120 uses a variety of pre-determined recipient-specific stimulation parameters/settings to convert the processed audio data into one or more sets of stimulation signals. These stimulation parameters include, for example, channel-to-electrode mappings, stimulation/pulse rate, pulse timing (electrical pulse width and inter-pulse gap), mode of stimulation (polarity, reference electrode), compression law or compression settings, amplitude mappings, etc. Amplitude mapping refers to the mapping of a sound intensity to a current level that is between the recipient's threshold (T) level (i.e., the level at which he/she can just hear the stimulus) and the maximum comfortable (C) level. In general, the stimulation parameters dictate how the processed audio signals are used for generation of sets of stimulation signals (current pulses) for delivery to the recipient. The human brain is organized into different areas of specialization that are each typically dedicated to relatively higher functions of brain activity. For example, the sensory areas of the brain (sensory brain areas) collectively refer to the region of the cerebral cortex that is associated with the receiving and interpreting of sensory information from various parts of the body. The sensory areas of the brain include somatic, auditory, visual, and olfactory cortical areas/regions. The auditory areas of the brain (auditory brain areas), in particular, are the parts of the brain that process sound information relayed thereto by the cochlea and auditory nerve.

Individuals who experience sensory deprivation will generally underutilize the sensory areas of their brain that are associated with the deprived sense. For example, an individual suffering from a hearing impairment may lack the ability to fully utilize the functional abilities of the auditory brain areas. The human brain is adaptable such that, when underutilization of a specific sensory area occurs, the brain will reorganize as a result of the underutilization. Some of the teachings detailed herein, such as those with respect to varying the cognitive load and otherwise managing the cognitive load on the recipient, are utilized to enhance or otherwise engage this adaptation, or at least enhance the rate of this adaptation relative to that which would be the case in the absence of such cognitive load variations.

For instance, congenitally blind research subjects have shown an enhanced ability to perform both auditory tasks, meaning that visual brain areas have reorganized for enhancement of the subject's hearing. This physiological phenomenon, sometimes referred to herein as cross modal brain reorganization or, more simply, cross modal reorganization, is most notably observed in early development (i.e., less than 7 years old) when neural plasticity is most prevalent, but this physiological phenomenon continues to a lesser degree into adulthood.

Although cross modal reorganization enhances behavioral performance for the recruiting modality, the cross modal reorganization also diminishes performance of the recruited modality. In other words, reorganizing from a less utilized brain area to enhance another sense negatively affects the less utilized brain area because it depletes the resources available to the less utilized area. For example, reorganizing the auditory brain areas in a sound deprived (deaf or partially deaf) individual in order to bolster visual ability results in greater auditory impairment. Therefore, if auditory information is later introduced through, for example, a cochlear implant, an individual who has experienced reorganization of the auditory brain areas as a result of their hearing impairment may find it more difficult to process the auditory information, when compared to an individual who has not experienced the same degree of cross modal reorganization. This is because the reorganized auditory brain areas have fewer or sub-optimally tuned cortical resources for use in processing the auditory information. Fewer cognitive resources results in a reduced cognitive capacity, which results in increased cognitive load and/or listening effort. In some exemplary embodiments of the teachings herein, cognitive load is managed, such as by increasing the cognitive load, so as to enhance or otherwise improve the rates of the cross modal reorganization so that in the long run, cognitive load is reduced in a given situation, all other things being equal.

Cross modal reorganization and associated deprivation of the auditory brain areas may manifest in different forms and occur for different reasons, leading to different auditory cognitive capacities/abilities in different recipients. As such, the performance of cochlear implants varies across the recipient population due, at least in part, to different cognitive auditory abilities. Accordingly, presented herein are techniques for selecting/determining stimulation parameters / operating parameters based on a recipient's auditory cognitive ability/capacity. In other words, the techniques presented herein determine cochlear implant stimulation parameters that are optimized/tailored to a recipient's unique cognitive auditory ability. Still further, presented herein are techniques for selecting/determining stimulation parameters / operating parameters to manage cognitive load, including, in some instances, increasing cognitive load. In other words, the techniques presented herein determine cochlear implant stimulation parameters that are optimized/tailored to increase cognitive load beyond that which would otherwise be the case based solely on the recipient's auditory cognitive ability/capability.

Accordingly, the below will be teachings directed towards establishing the recipients auditory cognitive ability/capability, after which will be described embodiments that can utilize that established ability/capability, to determine how to increase the cognitive load beyond that which would otherwise be the case.

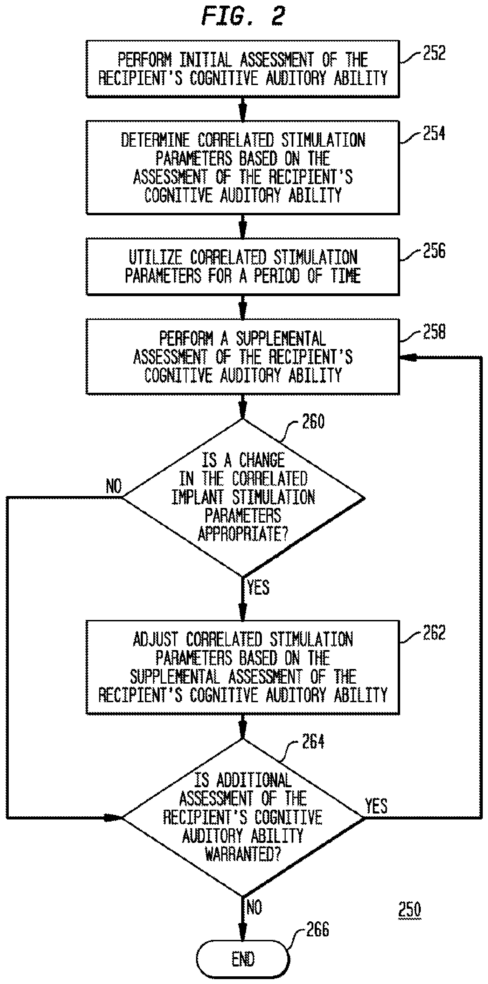

FIG. 2 is a flowchart of a detailed method 250 in accordance with embodiments presented herein. For ease of illustration, method 250 is described with reference to cochlear implant system 100 of FIG. 1.

Method 250 begins at 252 with the initial assessment of the cognitive auditory capacity/ability of a cochlear implant recipient. There are, in general, two methodological classes of tests/evaluations used to identify cognitive auditory ability, namely objective evaluations and subjective evaluations. Therefore, the initial assessment of the recipient's cognitive auditory ability may include one or more objective and/or subject evaluations that generate information useable to identify cross modal reorganization and to determine an estimated impact of the cross modal reorganization on a recipient's hearing capabilities.

Objective evaluations of cognitive auditory ability include, for example, functional near-infrared spectroscopy (fNIRS), functional magnetic resonance imaging (fMRI), Magnetoencephalography (MEG), Electroencephalography (EEG), etc. of a recipient's brain to evaluate the recipient's auditory brain configuration. More specifically, an imaging system generates results useable to characterize dynamic glucose metabolism, and thus metabolic activity, in different cortical areas in response to different sensory activities. For instance, the uncompromised auditory brain areas (i.e., auditory areas of the brain that have not experienced cross modal reorganization) will demonstrate increased metabolic activity when the recipient performs listening tasks/exercises. Conversely, if the auditory areas of the brain have experienced significant cross modal reorganization to support the visual system, the auditory areas of the brain will demonstrate increased metabolic activity when the recipient performs visual tasks, but little to no activity while performing purely auditory tasks. The degree of metabolic activity detected in response to different types of stimuli (e.g., auditory, visual, etc.) is used to objectively quantify how the auditory areas of the brain have been affected by cross modal reorganization (i.e., determine how much of the auditory areas of the brain are used by other non-auditory brain functions).

When fewer dedicated resources are available for the cognitive task of listening, listening tasks becomes more difficult or taxing for a recipient. As such, subjective evaluations of cognitive auditory ability may involve assessment of cognitive load or listening effort to highlight reduced auditory cognitive capacity or increases in other sensory modalities. As will be described below, in some embodiments, this assessment of cognitive load can be utilized as a baseline to determine whether or not operating parameters and/or maps should be adjusted so as to increase cognitive load. In one embodiment, the working memory of a cochlear implant recipient is assessed with a reading span task and a digit span task. The reading span task is a dual task paradigm in which subjects are asked to read printed sentences aloud and remember the last word of each sentence for later recall in the order presented. Following each sentence, the subject states whether the sentence is true or false. Sets range from 2-6 sentences in length, and at the end of each set, the subject is asked to recall the last word of each sentence. The forward or backward digit span tasks measures working memory in a similar fashion. A subject repeats lists of digits spoken in live-voice at a rate of one digit per second. The forward span task requires simply repeating back the series of digits, while the backward span task requires repeating the digits in reverse order. Two lists are presented beginning with a length of 2 digits and increasing in length by 1 digit after a successful repetition of at least one list at a given length.

It has been observed that there is a high coincidence of hearing problems and other sensory issues. As such, certain embodiments also include specific sensory tests/evaluations in the assessment of a recipient's cognitive auditory ability. Sensory evaluations, which are subjective in nature, may take a number of different forms, but are primarily designed to provide an understanding of functional sensory difficulties experienced by a recipient. Such an understanding may be important because recipients with different sensory issues are uniquely impacted in different sensory environments. In general, the sensory evaluations involve the elicitation and observation of a recipient's responses to different sensations and looking for evidence of difficulty making proper use of a sensory input. In general, an initial assessment of a recipient's cognitive auditory ability will include both objective and subjective evaluations performed in a clinical setting. For example, a medical practitioner (e.g., doctor, audiologist, clinician, etc.) performs an EEG on the cochlear implant recipient to objectively determine the extent of cross modal reorganization. Within the same timeframe (e.g., during the same one or two week window), the cochlear implant recipient is given a cognitive load test (e.g., a reading span rest), and perhaps a sensory evaluation. As will be detailed below, in an exemplary embodiment, this can be utilized as a baseline from which to determine when it is utilitarian to make adjustments to increase the cognitive load on the recipient.

The results of the objective and subjective evaluations are correlated with one another to generate a recipient's "auditory ability profile." As used herein, a recipient's auditory ability profile represents an estimated impact of the cross modal reorganization on the recipient's ability to process information received from via stimulating auditory prosthesis. In other words, the cross modal reorganization is analyzed in conjunction with the measures of cognitive load or listening effort to assess how the recipient's auditory brain areas are able to process electrical audio information which, as described further below, enables a determination of how stimulation parameters should be selected. According to the teachings detailed herein, the stimulation parameters are set, in some embodiments, so as to provide a cognitive load that is conducive to improving hearing, and no more. Conversely, according to some of the other teachings detailed herein, the stimulation parameters are set, sometimes, so as to provide a cognitive load that may in fact actually frustrate the recipients ability to hear, if only in a minor manner, relative to another set of stimulation parameters/operating parameters for that person's cognitive ability. Indeed, according to some of the other teachings detailed herein, stimulation parameters are set, in some embodiments, so as to provide a cognitive load that enhances or otherwise exercises the recipient so that the rate of adaptation due to the plasticity of the brain with respect to habilitation and/or rehabilitation is enhanced relative to that which would otherwise be the case.

Returning to the specific example of FIG. 2, at 254 the results of the initial assessment of the recipient's cognitive auditory ability, represented in the recipient's auditory ability profile, are analyzed and used to determine stimulation parameters for use in the recipient's cochlear implant 104. As noted above, the recipient's stimulation parameters are the settings/parameters that dictate how the stimulator unit 120 of the cochlear implant 100 will convert the processed audio data into stimulation signals for delivery to the recipient's cochlea 130. The stimulations signals generated and delivered to the recipient's cochlea 130 operate as a form of electrical audio information that is presented to the recipient's auditory brain areas via the cochlea nerve cells. The various stimulation parameters control the amount of electrical audio information that is presented to a recipient at any given time.

As a result of cross modal reorganization and other factors, the auditory cortices of different recipients have different abilities to process electrical audio information. Therefore, in accordance with the embodiments presented herein, the recipient's auditory ability profile is analyzed to select stimulation parameters that will generate electrical stimulation signals that, when delivered to the recipient, optimize the amount of electrical audio information presented to the recipient in view of the recipient's cognitive auditory ability (i.e., match/correlate a measure of the information expected to be presented by the stimulation parameters to the estimated ability of the recipient's auditory brain areas to process electrical audio information). In other words, the stimulation parameters are correlated to, and selected based on, the recipient's cognitive auditory ability. As used herein, stimulation parameters that are selected based on the recipient's cognitive auditory ability are referred to herein as "correlated stimulation parameters." Correlated stimulation parameters currently in use by a cochlear implant are sometimes referred to herein as the "current" or "present" correlated stimulation parameters.

At 254, the initial correlated stimulation parameters are customized for the recipient based on the recipient's cognitive auditory ability. This as the results of providing stimulation parameters that enhance hearing and render the recipient comfortable when utilizing the prostheses. This reflects a potential advantage over conventional techniques use for selection of a recipient's initial stimulation parameters. More specifically, as noted above, there are a large number of stimulation parameters that can be selected for a cochlear implant recipient. In conventional techniques, an audiologist selects initial stimulation parameters for a recipient based on, for example, the audiologist's clinical knowledge, parameters/programs known to be useful for other cochlear implant recipients, study results, etc. Since, in such conventional arrangements, the stimulation parameters are not at all customized to the recipient, there is a significant possibility that the selected stimulation parameters will not be acceptable to the recipient. As such, after selection of initial stimulation parameters, in conventional arrangements the audiologist and recipient undertake a time-consuming, expensive, and difficult trial-and-error process in which sounds are delivered to a recipient and, using verbal feedback, the audiologist evaluates and changes/adjusts the initial stimulation parameters. Although difficult for many recipients, such trial-and-error processes are unworkable with young children since they lack the ability to provide the necessary feedback to the audiologist.

However, as noted above, in accordance with the embodiments presented herein, the initial correlated stimulation parameters are customized for the recipient based on the recipient's cognitive auditory ability (only the ability, as opposed to other embodiments detailed below, where the initial correlated stimulation parameters are customized for the recipient based on both the recipient's cognitive auditory ability, and based on a training regime/habilitation regime/rehabilitation regime, which will often result in the stimulation parameters being parameters that impart additional cognitive load on the recipient beyond that which would otherwise be the case. Either way, there is a greater likelihood that the initial correlated stimulation parameters selected at 254 will be suited for the recipient. This reflects an advantage in that the trial-and-error processes can be eliminated (e.g., for young children) or substantially reduced since the audiologist is unlikely to have to initial significant parameter changes. Returning to the example of FIG. 2, after the selection of the recipient's initial correlated stimulation parameters at 254, at 256 the recipient is allowed to use the correlated stimulation parameters for a period of time. In one example, the correlated stimulation parameters are used during several hearing tests conducted over a short period of time before performance of the supplemental assessment of the recipient's cognitive auditory ability (e.g., a shortened parameter evaluation process with the audiologist). In other embodiments, the recipient is allowed to use the correlated stimulation parameters over a longer period of time (e.g., several days or weeks).

At 258, a supplemental assessment of the recipient's cognitive auditory ability is performed. The supplemental assessment of the recipient's cognitive auditory ability may take a number of different forms, but generally includes one or more subjective as described above with reference to the initial assessment of the recipient's cognitive auditory ability. In accordance with embodiments presented herein, the supplemental assessment may be performed in a clinical setting or, in certain examples, in the recipient's home or other remote (i.e., non-clinical) setting. For example, at a follow-up clinical appointment, subjective evaluations are performed to identify changes in functional cognitive capacity (cognitive load). The results of the supplemental assessment are used to update the recipient's auditory ability profile (i.e., correlated with the results of previous subject and objective evaluations). Because some embodiments herein are directed towards increasing the cognitive load on the recipient that results from utilizing the hearing prostheses, the supplemental assessment can be used to determine whether or not the operating parameters and/or maps should be adjusted to increase the cognitive load.

Supplemental assessments may include objective evaluations (e.g., imaging), but, in general, objective evaluations would be repeated less frequently as they are more time-consuming and potentially require more resources than subjective cognitive load evaluations. Nonetheless, correlations between the objective and subjective evaluations are checked and realigned periodically.

In certain embodiments, the supplemental assessments include subjective evaluations (e.g., cognitive load testing) that are performed in a remote (i.e., non-clinical) environment via, for example, a smart phone, computer, or other consumer electronic device. In such examples, as long as the remotely performed subjective evaluations are correlated/aligned with the subjective evaluations performed in the clinical environment, it may be possible for a recipient to initiate changes in stimulation parameters based on self-evaluations of auditory ability.

The supplemental assessment (or possibly the initial assessment) may be accompanied by one or more hearing tests that objectively (e.g., Neural Response Telemetry tests relying upon electrically evoked compound action potential (ECAP) measurements) or subjectively (e.g., verbal feedback tests) evaluate the performance of the cochlear implant. Such tests are not designed to evaluate cognitive auditory ability, but instead attempt to determine how well the current correlated stimulation parameters are working for the recipient (i.e., is the recipient, when using the correlated stimulation parameters, able to understand the information that is presented at each stimulation cycle). These hearing tests may be useful in determining, for example, whether the recipient is having difficultly hearing in general, difficulty in noise, difficulty in certain frequency ranges, etc.).

After performance of the supplemental assessment of the recipient's cognitive auditory ability, at 260 the current correlated stimulation parameters are evaluated to determine if a change in the correlated stimulation parameters is appropriate. That is, the results of the supplemental assessment (i.e., the recipient's updated auditory ability profile) are utilized, possibly in conjunction with the results of the one or more other hearing tests, to determine if the correlated stimulation parameters currently in use by the cochlear implant 104 are properly correlated to the recipient's cognitive auditory ability.

Because cognitive changes, identified by subjective evaluations (task load), objective evaluations, or both, reflect the increasing or decreasing ability of cognitive resources to adequately process the information presented by the cochlear implant, 260 of FIG. 2 represents a determination of whether there has been a long-term change in the recipient's auditory ability so as to warrant a change in stimulation parameters. If, at 260, it is determined that no changes should be made to the correlated stimulation parameters, then method 250 proceeds to 264, the operations of which are described further below. However, if it is determined at 260 that a change to the correlated stimulation parameters is warranted, then method 250 proceeds to 262 where the correlated stimulation parameters are adjusted.

More specifically, at 262, the correlated stimulation parameters are adjusted to either increase or decrease the amount of information presented through the use of the stimulation parameters. In other words, the stimulation parameters may be made more or less "information intensive" by, for example, changing the rate of stimulation, changing the number of spectral maxima, adjusting the Spectral Masking Threshold, adjustment of the Temporal Masking Offset.

Certain stimulation parameters have been proven to cause greater hearing difficulties than other parameters. For instance, presenting electrical stimuli to the cochlea in a more spectrally/physiologically dense configuration is generally more difficult for the recipient to process than more sparse presentations with a better spectral contrast. Although a dense presentation provides more information to the recipient, not all recipients are capable of efficiently benefiting from it. Similarly, higher stimulation rates, although providing more information, have also been associated with listening difficulty among certain recipient (e.g., recipients often prefer programs with slower stimulation rates and fewer number of maxima) when listening to music (which can be considered as structured noise). Competitive auditory inputs (e.g., multiple speakers, music, noise, etc.) may also contribute to the creation of difficult listening conditions if not properly mitigated by stimulation parameter selection. For instance, noise reduction schemes that reduce competitive/confounding inputs are generally easier for listening because information overload is suppressed.

Therefore, in certain examples, reducing the rate of stimulation and/or the number of spectral maxima reduces the amount of information that is delivered to a recipient in each stimulation cycle while, conversely, increasing the rate of stimulation and/or the number of spectral maxima increases the amount of information that is delivered to a recipient in each stimulation cycle. The above adjustments are illustrative and it is to be appreciated that other adjustments to the stimulation parameters are possible in order to increase/decrease the amount of information that is delivered to a recipient in each stimulation cycle.

Similarly, recipients with different sensory issues perform differently in different sensory environments. Selecting stimulation parameters so as to mitigate the factors to which these recipients are sensitive will improve listening. For instance, if a recipient with sensory issues, particularly one who is sensitive to loud noises or noise in general, would likely benefit from a program that includes limits to loud amplitudes or includes noise reduction in general.

After adjustment of the correlated stimulation parameters, method 250 proceeds to 264 to determine whether or not additional assessment of the recipient's cognitive auditory ability is warranted. If additional assessment is warranted, the method 250 returns to 256 or 258 where the recipient is allowed to use the correlated stimulation parameters for a period of time before repeating steps 258-264. If it is determined that additional assessment of the recipient's cognitive auditory ability is not warranted, then method 250 ends at 266.

As noted above, individuals with hearing impairments are susceptible to cross modal reorganization that detrimentally affects the auditory areas of the brain. However, the introduction of auditory inputs, such as with electrical audio information provided via a cochlear implant, can reclaim some of the recipient's previously reconfigured auditory resources and can therefore increase the recipient's cognitive auditory ability. Therefore, not only does hearing performance decline with age, but it is also possible for a recipient's hearing to improve as the brain adapts to the stimulation. As such, understanding long-term cognitive auditory ability changes (i.e., changes over weeks, months, or even years) makes it possible to prescribe dynamic stimulation strategies and/or parameters. For example, if a recipient shows that cognitive ability is on the rise, then the recipient may be able to use more aggressive stimulation parameters (e.g., higher rates, greater spectral density, increased dynamic range, etc.).

Accordingly, in addition to tailoring stimulation parameters to the recipient's present cognitive auditory ability and sensory status, it is also possible to generate a rehabilitation strategy for the recipient. For example, a recipient may be prescribed with dynamic stimulation parameters that adapt over time to provide increasingly dense spectral representations of sound in an adaptive manner designed to challenge and improve the recipient's cognitive auditory ability. In other words, the stimulation parameters may be scheduled to periodically, randomly, progressively, etc. push the limits of how much information the recipient can process, so as to recover additional resources of the auditory brain areas.

In certain examples, the rehabilitation strategy may involve auditory training sessions or periods that are specifically designed to challenge the recipient and improve auditory function. Again, these rehabilitation sessions are configured such that the auditory training tasks that occur therein are modified in accordance with the recipient's auditory cognitive ability and may be updated as the auditory ability improves.

When performing rehabilitation, the recipient's response to auditory training sessions or periods may be monitored over a period of time to see if any improvement has occurred. If improvement is detected, stimulation parameters that provide a greater amount of information may be selected.

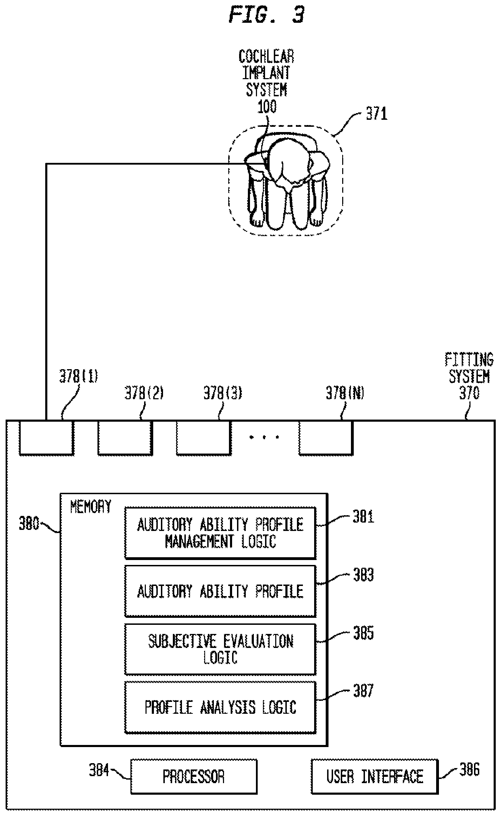

As noted above, method 250 includes both an initial assessment of a recipient's cognitive auditory ability and one or more supplemental assessments of a recipient's cognitive auditory ability. Also as noted above, the initial assessment of a recipient's cognitive auditory ability is generally performed in a clinical environment since the initial assessment includes both objective and subjective evaluations. FIG. 3 is block diagram illustrating an example fitting system 370 configured to execute the techniques presented herein.

Fitting system 370 is, in general, a computing device that comprises a plurality of interfaces/ports 378(1)-378(N), a memory 380, a processor 384, and a user interface 386. The interfaces 378(1)-378(N) may comprise, for example, any combination of network ports (e.g., Ethernet ports), wireless network interfaces, Universal Serial Bus (USB) ports, Institute of Electrical and Electronics Engineers (IEEE) 1394 interfaces, PS/2 ports, etc. In the example of FIG. 3, interface 378(1) is connected to cochlear implant system 100 having components implanted in a recipient 371. Interface 378(1) may be directly connected to the cochlear implant system 100 or connected to an external device that is communication with the cochlear implant systems. Interface 378(1) may be configured to communicate with cochlear implant system 100 via a wired or wireless connection (e.g., telemetry, Bluetooth, etc.).

The user interface 386 includes one or more output devices, such as a liquid crystal display (LCD) and a speaker, for presentation of visual or audible information to a clinician, audiologist, or other user. The user interface 386 may also comprise one or more input devices that include, for example, a keypad, keyboard, mouse, touchscreen, etc.

The memory 380 comprises auditory ability profile management logic 381 that may be executed to generate or update a recipient's auditory ability profile 383 that is stored in the memory 380. The auditory profile management logic 381 may be executed to obtain the results of objective evaluations of a recipient's cognitive auditory ability from an external device, such as an imaging system (not shown in FIG. 3), via one of the other interfaces 378(2)-378(N). In certain embodiments, memory 380 comprises subjective evaluation logic 385 that is configured to perform subjective evaluations of a recipient's cognitive auditory ability and provide the results for use by the auditory ability profile management logic 381. In other embodiments, the subjective evaluation logic 385 is omitted and the auditory profile management logic 381 is executed to obtain the results of subjective evaluations of a recipient's cognitive auditory ability from an external device (not shown in FIG. 3), via one of the other interfaces 378(2)-378(N).

The memory 380 further comprises profile analysis logic 387. The profile analysis logic 387 is executed to analyze the recipient's auditory profile (i.e., the correlated results of the objective and subjective evaluations) to identify correlated stimulation parameters that are optimized for the recipient's cognitive auditory ability.

Memory 380 may comprise read only memory (ROM), random access memory (RAM), magnetic disk storage media devices, optical storage media devices, flash memory devices, electrical, optical, or other physical/tangible memory storage devices. The processor 384 is, for example, a microprocessor or microcontroller that executes instructions for the auditory profile management logic 381, the subjective evaluation logic 385, and the profile analysis logic 387. Thus, in general, the memory 380 may comprise one or more tangible (non-transitory) computer readable storage media (e.g., a memory device) encoded with software comprising computer executable instructions and when the software is executed (by the processor 384) it is operable to perform the techniques described herein.

The correlated stimulation parameters identified through execution of the profile analysis logic 387 are sent to the cochlear implant system 100 for instantiation as the cochlear implant's current correlated stimulation parameters. However, in certain embodiments, the correlated stimulation parameters identified through execution of the profile analysis logic 387 are first displayed at the user interface 386 for further evaluation and/or adjustment by a user. As such, the user has the ability to refine the correlated stimulation parameters before the stimulation parameters are sent to the cochlear implant system 100.

The general operations for analysis of the recipient's auditory profile to identify correlated stimulation parameters that are optimized for the recipient's cognitive auditory ability have been described above. However, it is to be appreciated that the profile analysis logic 387 may operate in accordance with one or more selected guidelines set by a user via the user interface 386. For example, a user may configure the stimulation parameters that may be adjusted or set limits for how a stimulation parameter may be adjusted.

Some embodiments detailed herein are directed towards utilizing cognitive load as the primary basis upon which to control the prostheses, at least with respect to a change in control regimes. While some of the methods detailed above have been described in terms of evaluating cognitive load as a sub-evaluation or where the evaluation is a secondary evaluation that factors into an overall evaluation of the auditory capability, in contrast, in the following embodiments, cognitive load is the primary basis for controlling the prostheses in a changeable manner. That is, while the embodiments detailed above entailed evaluating cognitive load, and utilized such as a secondary feature to assist in the overall evaluation of the auditory cognitive ability/capability, in the following, the cognitive load is the primary feature under evaluation. Briefly, it is noted that cognitive load is differentiated from an auditory capability or auditory ability. In this regard, capabilities and abilities are features of how one performs. Put in terms of a utilization of a hearing prostheses, someone at the beginning of their hearing journey will perform less well than that same person will perform later on, such as six months, a year, two years, three years, etc., into their hearing journey. Conversely, cognitive load is something that will exist at all locations on that hearing journey and exists in some instances apart from the recipient's ability. Cognitive load is something that can increase with respect to better auditory capability or can actually decrease with respect to better auditory capability. Teachings detailed herein are in some instances directed towards applying a level of cognitive load over a long term temporal period, which cognitive load forces the recipient to develop mentally so that for all things being equal, the recipient's auditory capability increases.

In this regard, it is noted that the perceived mental effort to process speech information can be different from one person to another person. During any given day, the mental effort of the same person used in understanding audible speech (i.e. something that makes sense) varies, and sometimes, by a great deal, depending on both internal (e.g. mental and/or physical - state of mind and health) and external (e.g. environmental) factors. These factors can contribute, individually or collectively, to what is called the cognitive load on the person - essentially the amount of "brain power" needed to understand and process the input sensory data. This is different from the cognitive auditory abilities detailed above, which is a longer-term feature that varies in a meaningful manner over a longer period of time. To be clear, cognitive load is a factor in the cognitive auditory ability. However, it is only one of many factors. If cognitive load was to be held constant, it could be ignored in the analysis of cognitive auditory ability. Put another way, a person who has the capability to run a full marathon will have certain capabilities, but an evaluation of that person's capabilities to run a full marathon or even a half marathon significantly different in the results if the person has already run 15 kilometers or if the person is carrying a briefcase for work or a computer bag. Accordingly, the aforementioned utilization of the evaluation of cognitive load seeks to discount the effects thereof in the overall assessment of the recipient's cognitive auditory ability. Here, the effect on the recipient from the recipient's past short-term efforts (analogous to having run 15 kilometers) or current burdens (analogous to carrying a briefcase) is utilized with the following teachings. Moreover, the effect on the recipient from his or her past efforts can be utilitarian with respect to improving the recipient's ability to perform after having experienced such efforts.

Back to cochlear implants. Hearing with a cochlear implant is a different sensorineural process than what the recipient would have previously experienced. For the cochlear recipients, it takes time for the brain to adapt and perceive sound through this new sensorineural pathway. A heavy cognitive load can lead to negative effects on task completion, including the ability to hear someone speak, that eventually impacts the speech intelligibility and speech recognition. People of certain demographics can experience more periods with higher amounts of cognitive load than others, such as, for example, children, elderly, etc. One example of the negative effects of high cognitive load can be a detrimental impact on their center of balance. In the context of cochlear implant recipients, cognitive load is a utilitarian factor to consider especially when they are early in their rehabilitation journey. The mechanism through which they hear, and the sensorial sensation of hearing is different, and this can impact significantly the cognitive load they experience in many (new) hearing situations. Through a customized tracking on their cognitive load and a system that seeks to maintain an optimal amount of cognitive load be utilitarian to improving their speech perception performance during their rehabilitation and help them to appreciate the full spectrum of sound through their cochlear implant system.

In some embodiments, measures of cognitive load are utilized as a metric to evaluate a success or a likelihood of success of a recipient's map. Embodiments herein include developing utilitarian maps having settings that improve the likelihood that the recipient does not need to strain too hard to hear (at least relative to compared maps) - meaning that the average cognitive load required by the recipient is reasonable (or at least less straining than other maps). Cognitive load is also used in some embodiments as a measure of the rehabilitation performance of a cochlear implant recipient. Initially, after switch on, the average cognitive load required for the recipient to hear would be high, as they adapt to the electrical-medium of hearing, as well as re-learn how to perceive sounds. Over time the brain will learn and adapt, however, so the cognitive load required will drop.

Embodiments include measuring or otherwise obtaining information indicative of the average cognitive load required for a recipient to hear, and utilizing such as a marker in the recipients rehabilitation. Cognitive load measures can be used to indicate when it is time to make modifications to the recipient's map so as to improve or otherwise enhance the rehabilitation journey relative to that which would otherwise be the case. This is sometimes referred to herein as the progressive maps concept. In some such embodiments, the teachings herein enable a determination, utilizing cognitive load measurements / data, as to when it is utilitarian to "progress" the recipient's map.

Some embodiments include a method of applying a first map (i.e., the first map that is applied as a result of a compete fitting session - the map that is utilized by the recipient to function in the real world after the recipient is fitted with the hearing prostheses, as opposed to an experimental map as compared to another experimental map utilized during fitting to determine which of the two maps should be utilized by the recipient to function in the real world) and then as the recipient adjusts to the device and the new electrical sensorineural means of hearing, the recipient's cognitive load in listening situations decreases on average. By way of example only and not by way of limitation, in an exemplary embodiment, effectively, their brain has adapted to the current map. (This is different from the aforementioned cross-modal adaptation. To the extent that the recipient has experienced cross-modal adaptation, the adaptation of the brain associated with becoming accustomed to the cochlear implant occurs in the portion of the brain that has been cross-modalized.) In an exemplary method, these cognitive load changes can be used as markers to then make map adjustments as appropriate for the rehabilitation process. By way of example only and not by way of limitation, in an exemplary embodiment, the map adjustments could correspond to increasing the dynamic range of the prosthesis, increasing the number of channels that are activated, etc. In some embodiments, these map adjustments correspond to any map adjustments that essentially provide more information to the brain to allow the brain to further adapt. In some embodiments, the stimulation pulse per second can be varied, a parameter in the map can be enabled in such a way that certain aspects of an algorithm will be unlocked or enhanced so that the recipient can hear the subtle differences in the audio under this new level. In some embodiments, the number of maxima (stimulation pulses per frame) can be increased, the frequency mapping/channel assignment can be changed and/or stimulation current steering (such as multipolar stimulation, where multiple electrodes are used in combination to achieve finer resolution current paths) can be used. Moreover, a more aggressive noise reduction algorithm can be used. The dynamic range of the device can be adjusted. For example, threshold levels and threshold SPL (the input acoustic level below which input levels are ignored) can be changed. A temporal masking decay threshold can be adjusted. Also, signal processing parameters, such as automatic gain control attack and release times can be changed. Any variable that can be adjusted to enable the goals herein can be used in some embodiments.

It is noted that map adjustments may be different from the temporary / real time adjustments to parameters of the prosthesis during normal operation thereof. By way of example only and not by way of limitation, in an exemplary embodiment, a beamforming technique could be applied that focuses the sound capture apparatus of the prosthesis to a location directly in front of the recipient upon a determination that the recipient is having difficulty comprehending speech (because, for example, too much background noise is being introduced), and, in some instances, upon a determination of the recipient is experiencing increased cognitive load at that time. The result of the beamforming can be to reduce the amount of background noise and to reduce the cognitive load on the recipient at that time improve the recipient's ability to hear or otherwise understand the words spoken to the recipient, again, at that time.

In an exemplary embodiment, stimulus can be provided to the recipient and/or a "scene" can be artificially created for the recipient to influence the recipient. For example, the recipient could be one who is subject to tinnitus under certain external environmental conditions (too quiet or too loud noise, etc.) and/or under certain internal body conditions (high blood pressure or a temporary disease) - this can, in some embodiments, not be tinnitus related - such conditions can also affect the overall ability of the recipient to comprehend speech. There can be a scenario is which the recipient is experiencing stress/anxiety because of the ringing or buzzing going on with his/her auditory nerves. This indirectly induces a higher than normal cognitive load on the person. The system can be configured to detect this or otherwise configured to receive input indicating that tinnitus is occurring (the recipient could say out loud - "I have the bad ringing in my ears again" - and the system would identify such as the tinnitus scenario. Upon the system determining that tinnitus is occurring, the system could be configured to play a relaxing-natural soothing sound (rain on water, for example) to help to calm down his/her stress/anxiety so as to alleviate his/her cognitive load. In this example, something is altered that is not map related. In an exemplary embodiment, the system could insert low level instrumental background noise (Kenny G ^{™} music, for example), or possibly soothing vocalists. It is noted that this can be subjective to the recipient. Some will find Rap and Metallica ^{™} soothing and find the works of Mozart and Bach infuriating. To each his or her own. Note the converse - in an exemplary embodiment, non-soothing music can be utilized to increase the cognitive load.

In some embodiments, mixing ratios - the relative levels at which audio source input is combined at the output (say from microphones and an auxiliary source, like wireless streaming) can be varied. In some embodiments, C-levels (maximum comfort levels) can be varied. In some instance, both T and C levels can be varied. In some instances, the variation can be weighted (e.g., more T level variation than C level (2 to 1, 3 to 1, 1.5 to 1, 4 to 1, etc.). In some embodiments, output volume could be adjusted. This could be more of a direct recipient's choice in some instances than other parameters.

Any parameter detailed herein can be changed or otherwise altered to implement the teachings detailed herein. Map adjustments are therefore adjustments to things that are more temporally stable. Whereas an adjusted map may be something that is used for more than a week, 2, 3, 4, 5, 6, 7, 8, 9, 10, 11 or 12 weeks, or 4, 5, 6, 7, 8, 9, 10, 11, 12, 13, 14, 15, 16, 17, 18, 19, 20, 21, 22, 23, or 24 months or more, and an adjusted parameter may be something that is utilized for minutes, hours, or maybe a day or two, or approaching a week at most. Again, consistent with the analogy of the marathon runner, someone that is capable of running a marathon might take action after running 15 km that that person might not otherwise do after running 1000 m. Such a person might choose to run in the shade after running 15 km whereas the person may not be concerned about running in the shade for only 1000 m. Conversely, in both situations, the runner is unlikely to change his or her pair of shoes. The point is, with reference to map adjustments, these are adjustments that relate to longer-term subjective capabilities of the recipient as opposed to short-term capabilities.

In this regard, in contrast to adjusting parameters on a scene or scenario basis (loud background noise, listening to music, listening to television, having to be attentive to oncoming traffic while walking, having to understand a specific speaker, becoming fatigued during a conversation or during the day, becoming energetic during a conversation or during the day, etc.), embodiments can include adjusting the map(s) to improve an overall rehabilitation journey, which is measured in terms of months, and years, to manage the cognitive load on the recipient, thereby using a map that results in limiting the information provided to the brain such that not so much information is provided to the brain that it is prevented from being able to learn, but also to avoid long term use of a map that underutilizes the recipient's brain, thereby avoiding a situation where the recipient's cognitive capacity is not under-utilized in the rehabilitation journey. By rough analogy, such is like nursery school, where the learning material progressively becomes harder for the child, but not too hard (e.g., shapes, colors, letters, numbers, are taught, followed by progression to limited reading, limited mathematics, but not division, percentages, young juvenile novels, etc., thereby providing a healthy cognitive load that causes progression, but preventing the cognitive load from being too high on a long term basis). This as opposed to a scenario where a child is having hard time for various immediate reasons and simply must be allowed to color with crayons (thereby reducing the cognitive load at that time, but the child is not permitted to color every day, and will eventually be forced to learn how to read, perform addition, subtraction, etc.) or a scenario where the child is on point for that day and is just ripping through material and thus can be provided with more difficult material for that day.

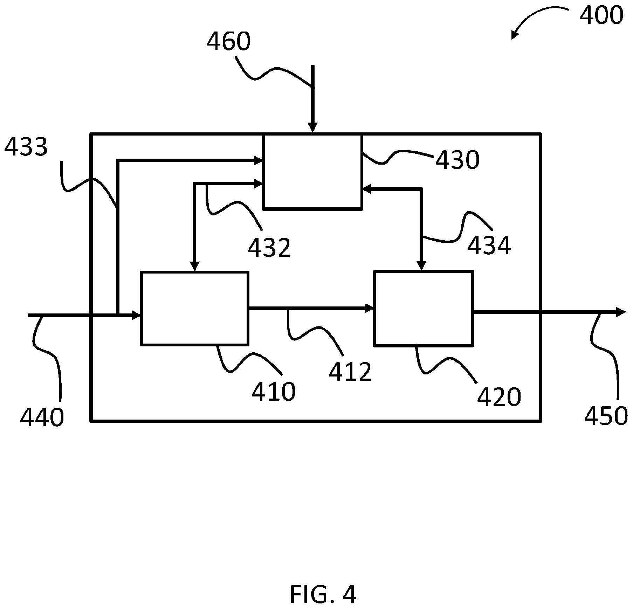

FIG. 4 presents a functional schematic of an exemplary prosthesis 400, or at least a portion thereof, according to an exemplary embodiment. In an exemplary embodiment, the prosthesis 400 corresponds to the retinal implant detailed above, while in other embodiments the prosthesis 400 corresponds to the cochlear implant detailed above with respect to FIG. 1. That said, in some alternate embodiments, the prosthesis is another type of prosthesis, such as by way of example only and not by way of limitation, a middle ear implant or a bone conduction device implant. In an exemplary embodiment, the prosthesis 400 includes a processor 410, which in an exemplary embodiment, can be a light processor and/or a sound processor. The processor 410 receives a signal 440 indicative of input that is based upon a captured physical phenomenon, such as sound, light, etc. The processor 410 processes the input 440, and outputs a signal 412 that is based on the processed input to tissue stimulator 420. In an exemplary embodiment, tissue stimulator 420 is configured to stimulate the tissue to evoke a hearing percept based on the signal 412. This is represented by arrow 450, which represents the output of stimulative energy to tissue of the recipient. In an exemplary embodiment, the output is an electrical signal, such as the case with respect to the output of an electrode of a retinal implant and/or a cochlear implant.

Prosthesis 400 further includes an input unit 430, which is configured to receive input indicative of a dynamic cognitive capability of a recipient. In an exemplary embodiment, input unit entails a toggle switch or the like that is configured so that the recipient can depress the switch so as to provide input, represented by input 460, into the input unit. The input unit 430 is in signal communication with the processor 410 via signal path 432. In an exemplary embodiment, the input unit 430 receives the input 460 from the recipient that indicates that the recipient is of a certain dynamic cognitive capacity (more on this below). In an exemplary embodiment, the input unit 430 receives the input 460 from the recipient indicating that the recipient wants the prosthesis to operate differently from that which it is currently operating, because, for example, the cognitive capability of the recipient has changed and/or because the recipient has become fatigued and/or because the sound and/or light that the recipient is receiving requires more cognitive effort specifically or effort in general to comprehend, all other things being equal. That said, as will be detailed below, input unit 430 further includes, in some embodiments, the capability to receive input indicative of latent variables or the like that are indicative of the recipient becoming fatigued, the recipient having less cognitive capability than that which was previously the case and/or that the sound and/or light to which the recipient is being exposed requires more effort to comprehend.

Briefly, still with reference to FIG. 4, it can be seen that the input unit 430 is in communication with the sound processor 410 via signal line 432, which will enable the input unit 430 to provide input to the sound processor so that the sound processor processes sounds in a different manner according to the teachings detailed herein, which corresponds to a different operating regime and/or different operating mode and/or operating the prosthesis differently. Also as can be seen from FIG. 4, the input unit 430 is in communication with the tissue stimulator 420 via signal line 434. In an exemplary embodiment, input unit 430 can communicate directly with the tissue stimulator 420, and, in some embodiments, control the tissue stimulator 420 so that the prosthesis 400 operates differently. That is, the input unit 430 bypasses the processor 410 and the input unit 430 in combination with the tissue stimulator 420 changes the operating regimes of the prosthesis 400. In an exemplary embodiment, this can entail the elimination of certain channels from being outputted by the tissue stimulator 420. In an exemplary embodiment, this can entail the prevention of energizement of one or more electrodes of an electrode array where the tissue stimulator 420 is a cochlear electrode array. It is noted that the input unit 430 can work with both the processor 410 and the tissue stimulator 420 at the same time to achieve any of the results detailed herein and/or variations thereof.

It is noted that in at least some exemplary embodiments, the input required to adjust these specific features could become voluminous. In this regard, in an exemplary embodiment, prosthesis 400 can be configured to communicate with a portable handheld device, such as by way of example, a so-called smart phone and/or a so-called laptop computer. Such devices can enable more ease of management and/or more ease of input of the various parameters that can be adjusted as detailed herein and/or other parameters that can be adjusted to account for fatigue and/or for varying cognitive capacity, and/or for input that requires more effort.

The embodiments detailed above have focused on recipient input as a conscious act into input 430. As noted above, in an alternate embodiment, the prosthesis can utilize latent variables to determine or otherwise indicate that the recipient is at a fatigue level and/or that the recipient has experienced a change in his or her dynamic cognitive capabilities and/or that a change has occurred in an environment that requires more effort to comprehend the given input relative to that which was the case, all other things being equal.

In an exemplary embodiment, the input into unit 430 can be utilized to "downshift" the prostheses so as to lower the cognitive load that results in the utilization of the prostheses, all other things being equal. The term "downshifting" is used herein to describe the changes to the operation of the hearing prosthesis. In this regard, the term "downshifting" is meant to mean that the prosthesis is operated in a manner such that the prosthesis operates in a less than optimal matter for conditions that would otherwise warrant the more optimized matter. In this regard, this differentiates from a scenario where, for example, a hearing prosthesis is changed from an omni directional mode to a beamforming or directional capture mode because that scenario warrants such operation. Conversely, downshifting would entail utilizing beamforming or directional capture mode even though the situation would otherwise not call for such, solely because the recipient has become fatigued and/or the recipient has experienced reduced cognitive capability. It is noted that the term "downshifting" as used herein and elsewhere in the art, corresponds to short-term changes to address short-term fatigue and/or cognitive fluctuations. This in a manner analogous to utilizing a vehicle at a lower gear setting for a specific reason. A long-term change would be analogous to devoting a car or truck to utilization on a steep mountainside where, for example, the car or truck would always be operated in first gear. Corollary to all of this is the concept of upshifting, which also corresponds to short-term changes to address short-term energy bursts and/or cognitive fluctuations. In some embodiments, the input into unit 430 can be used to upshift the prosthesis so as to increase the cognitive load that results from utilization thereof, all other things being equal.

Still further, downshifting (and thus upshifting) is directly tied to the current state of the recipient, whether that is an affirmative input by the recipient or a determination by the prosthesis based on latent variables or the like. To be clear, this differentiates from establishing or otherwise operating the prosthesis in a given operating regime because the recipient has that specific cognitive capability on a long-term basis.

In contrast to the embodiment of figure 4, an alternate embodiment utilizes or otherwise relies upon a more long-term approach to cognitive load, hereinafter referred to as average cognitive load. In this regard, the teachings above associated with figure 4 can be considered instantaneous or short-term cognitive load / instant cognitive load. Conversely, the average cognitive load is more of a long-term cognitive load. Put another way, the average cognitive load would factor out or otherwise somehow statistically accommodate the short-term cognitive load swings due to fatigue. In this regard, while the upshifting and/or downshifting of the prosthesis detailed above could be based on cognitive loads determined for periods of time lasting minutes, hours, or maybe a day or two (e.g., owing to some illness or mental stress, for example), the average cognitive loads is indicative of the cognitive load spanning many days, spanning weeks, months, or a year or two or more.

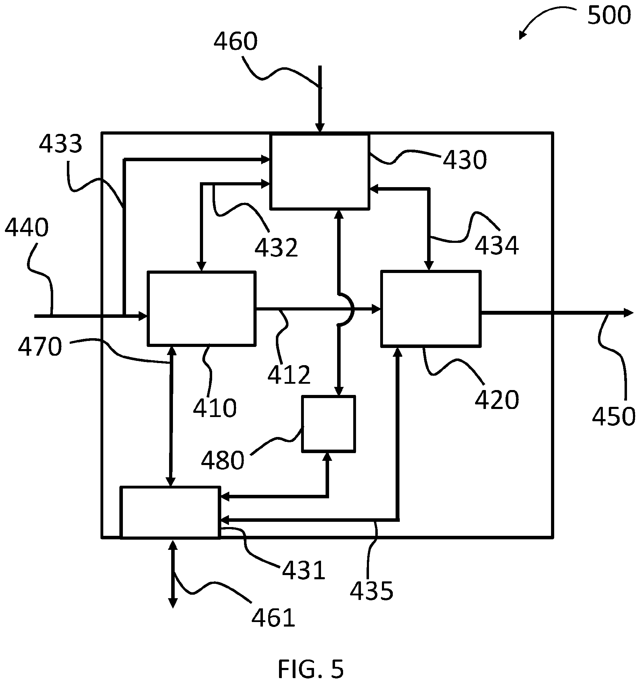

FIG. 5 presents an alternate embodiment of the prosthesis detailed in figure 4, which is configured for utilization based on the average cognitive load concept (as well as, in some embodiments, the aforementioned short-term/instantaneous cognitive load, steps of figure 4). Here, prosthesis 500 includes the features of prosthesis 400 vis-à-vis the like reference numbers as can be seen. Also as can be seen, there is a second input unit 431, which receives second input 461. Briefly, it is noted that input unit 431 is in signal communication with processor 410 via input line 470, and with the tissue stimulator via signal line 435. Also, unit 431 is an input-output unit, as represented by the double arrow 461. Input-output unit 431 is in signal communication with memory 480. Memory 480 is also in signal communication with input unit 430. While not shown, in some embodiments, memory 480 can be in signal communication with processor 410 and/or tissue stimulator 420. In an exemplary embodiment, memory 480 records input into input 430 and/or records inputs into input-output unit 431 and/or output out of the input-output unit 431.

In an exemplary embodiment, input unit 430 includes the ability to receive input from biometric assessment units that are indicative of the recipient's cognitive load. Alternatively, and/or in addition to this, input-output unit 431 also has this capability. In this regard, prosthesis 500 provides a mechanism that utilizes real-time biometrics assessment of the individual's cognitive load, which is logged (e.g., in memory 480) and then used during fitting at the clinic, to configure the map and device settings to influence the recipients rehabilitation journey.

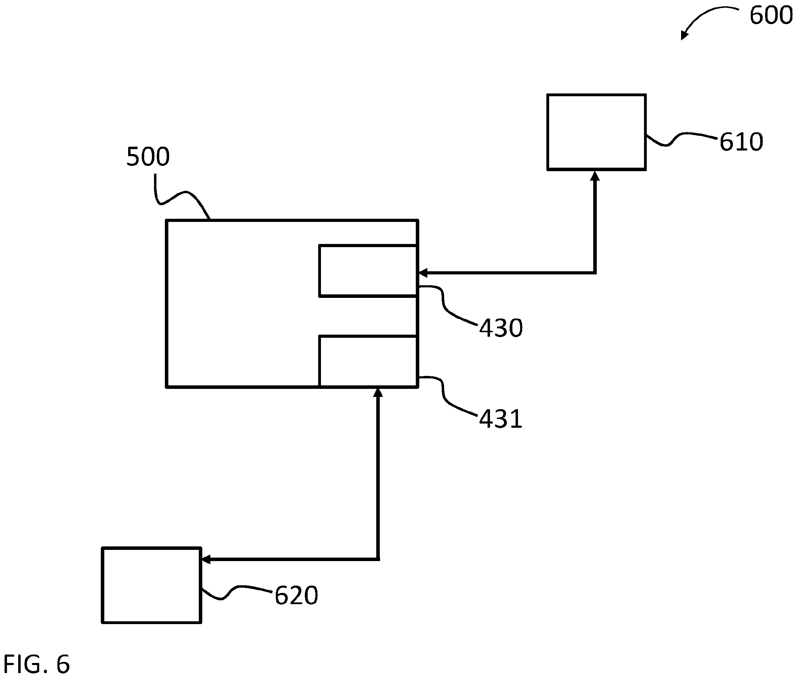

In an exemplary embodiment, there is a system 600, as shown in FIG. 6, that includes a system of sensors 610 configured to track and/or monitor the responses of a person (e.g., pupillary response (a linear relationship is believed to be present with the increase in pupil dilation as the demands a task places on the working memory increase), speeding heart rate, breathing rate, and/or release of certain hormones like adrenalin and cortisol (that can indicate a sudden stress, anxiety, fear or nervousness). These measures can be used by the prosthesis or another device to estimate/determine the cognitive load on the person.

The input from these sensors 610 is received by input unit 430. These are provided to memory unit 480 in some embodiments in a manner of raw signal where they are recorded. Alternatively, and/or in addition to this, the signals are provided to the processor 410, which processes these signals or otherwise refines the signals, and the processed and/or refined signals are then provided to memory 480. That said, a separate processor or otherwise is module of the prostheses can refine or otherwise process the signals. Accordingly, in an exemplary embodiment, the system 600 can also include a module that can analyze these biometric signals recorded from these sensors in real-time and use this information to form a mental state of that individual with respect to his/her present cognitive load or otherwise to form or produce data indicative of the cognitive load of that individual. In any event, this information is stored in memory 480 so it can be used later (such as in a clinic environment - more on this below).

It is briefly noted that while the embodiment of figures 5 and 6 indicate that the memory is part of the prosthesis 500, in some other embodiments, the memory can be a remote device in the prosthesis, such as a portable consumer electronics device, such as a smart phone or the like. In this regard, in an exemplary embodiment, the prosthesis 500 can be in communication with this portable electronics device, whether via a wireless signal or otherwise, such that the information can be stored.

System 600 further includes unit 620. In an exemplary embodiment, unit 620 is a module that is configured to process the cognitive load data (whether in the raw form or the processed form or both) and advise on map adjustments so as to adjust the rehabilitation journey based on the maintenance of a temporally significant utilitarian cognitive load (as distinguished from, for example, the decrease to an instantaneous or short-term cognitive load as detailed above with respect to figure 4). In an exemplary embodiment, unit 620 is a remote unit that is remote from the prosthesis 500. In an exemplary embodiment, unit 620 is or includes a processor. Unit 620 can be a personal computer or portable computer or the like that is programmed to process the cognitive load data and develop map adjustments. In an exemplary embodiment, unit 620 is a smart phone or some other consumer electronics device. Alternatively, unit 620 is in signal communication with the prostheses 500 and/or the aforementioned remote device that serves as an alternate and/or a backup to the memory 480 via the Internet or a phone connection, etc.

It is briefly noted that while the embodiment of figure 6 depicts the sensors 610 and the unit 620 as remote components from the prostheses 500, in some alternate embodiments, one or both are integral components of the prostheses.

In some embodiments, any mechanism of measuring and/or evaluating cognitive load of an individual while he/she is performing a listening and/or a viewing task can be used. For example, Brünken, Plass, and Leutner methods can be used, such as those detailed in "Direct Measurement of Cognitive Load in Multimedia Learning", EDUCATIONAL PSYCHOLOGIST, 38(1), 53-61 (Brünken, Roland; Plass, Jan L.; Leutner, Detlev (2003)). For example, the methods can be classified into two groups: objectivity (subjective or objective) and causal relation (direct or indirect). Overall, these methods could be: self-reported mental effort/stress level/difficulty of materials, physiological measures, behavioral measures, brain activity measures, etc. Alternatively, and/or in addition to this a psychometric component, eye tracking component, Electroencephalography (EEG) component and/or statistical analysis, etc. can be used. For example, any method disclosed in "Measuring cognitive load in the presence of educational video: Towards a multimodal methodology", Australasian Journal of Educational Technology, 2016, 32(6) (Kruger, Jan-Louis; Doherty, Stephen (2016)) can be used sometimes.

In an exemplary embodiment, one or more of respiration rate, heart rate, brain activity, skin temperature, perspiration, blood pressure, perspiration composition, saliva composition, blood composition, breath composition, eye movement, pupil dilation, blink rate, length of eyelid closure time, limb and/or head movement and/or digit movement, and/or rates, in some instances compared to a baseline, can be used to evaluate or otherwise measure cognitive load. In at least some exemplary embodiments, system 600 includes one or more sensors configured to detect or otherwise measure or otherwise receive input indicative of the aforementioned physical phenomenon. Indeed, automated sensors can be utilized that measure one or more or all of the above phenomenon. Alternatively, and/or in addition to this, observation by another human can be utilized to collect the data. In this regard, in at least some exemplary embodiments, the measurements of cognitive load can be presented in a classroom and/or a clinical setting, as the goal of at least some exemplary embodiments is to increase the cognitive load on a long-term basis, and thus the clinic and/or classroom setting results can be utilized in a manner analogous to evaluating a student's performance on a test to determine that he or she should move on to the next grade level.

One or more or all of the aforementioned biometrics can be obtained in real time. The evaluation thereof by the analysis module /processor can occur in real-time or otherwise can occur after the data is collected. In an exemplary embodiment, data can be extracted from the memory 480 and then analyzed.

Corollary to the above is that while the embodiment of figure 6 depicts the sensors in signal communication with the prosthesis, in some alternative embodiments, the sensors are in communication (signal or otherwise) with a fitting device or another remote device or the like. That is, while the embodiment of figure 6 has been presented as a prosthesis centric device, in some alternative embodiments, components 610 and 620 are completely separate from the prosthesis. In a very simple example, in an exemplary embodiment, a video camera can be utilized to record the recipient's actions. The recipient's actions, such as head movement, eye movement, blink rate, movement of head and/or movements of arms, body posture, etc., can be evaluated by a professional who reviews the camera. Note also that a series of accelerometers or sensors can be utilized to non-visually track the movements of the recipient's body.

In an exemplary embodiment, one or more than one or all of the biometric data components can be analyzed and collectively provide an overall picture of the state of mind and/or health related to cognitive load of the recipient during a given period. It is noted that in at least some exemplary embodiments, some of the biometric data can be discounted on an individual instance basis and/or on a permanent basis (e.g., a person who is blind might not have meaningful eye movements ever, but a person who is suffering from allergies at a given time may not have meaningful eye movements during that given time, but might at a later date).

In some embodiments, based on the measured biometric data, a result corresponding to the overall cognitive load state of the individual can be determined. Based on this result, enhanced configuration/settings of the user map/hearing device could be stored, trained, and compared in such a way that when a high cognitive burden is detected, the system would intelligently adjust the prosthesis by loading a special setting from the prosthesis, or some other device, such as a personal assistant, and/or from a database (e.g., from the cloud) so that the cognitive load on the person is reduced without significantly impacting their hearing performance. Also, in a similar vein, if the cognitive load is determined to be low, based on analysis of the aforementioned result, the prosthesis or system thereof could adjust map settings in order to present a wider range of stimuli to the recipient.

Thus, some embodiments provide devices, systems, and/or methods that account for the fact that, at different stages of the rehabilitation journey, recipients are trying to adapt and, in some instances, re-learn (rehabilitate, as opposed to habilitate) to hear or see and understand the sound / light through the new pathway resulting from the prosthesis. Starting with and setting up with the right amount of cognitive load on the individual to re-learn can be utilitarian. By way of example only and not by way of limitation, a heavy cognitive load could potentially have a long lasting impact on negative learning. As the individual starts to get himself/herself familiar with the listening / the seeing via, for example, the cochlear implant or the bionic eye, the load can potentially be increased or brought up to the level that would correspond to a maximum permissible / utilitarian value at that instance. With this new level of load, for example, new map settings (i.e. progressive maps) and/or training materials would be applied. In some embodiments, it can be seen that this can be an iterative process. Indeed, in some instances, as the load is increased, it is possible to "overshoot" the maximum permissible/utilitarian value at that instance. In this regard, in at least some embodiments, such will be a matter of course, in view of the fact that the systems utilize a feedback loop that receives one or more of the aforementioned inputs relating to one or more of the aforementioned biometric phenomenon and evaluates that input to determine the cognitive load at that time, and thus upon an indication that the cognitive load has been increased to high, the cognitive load can be decreased, after which the process is repeated, and so on. It is possible that there could be a scenario where there is an increase, an increase, an increase, a decrease, an increase, a decrease, and then an increase, where the last increase corresponds to the cognitive load that will be inflicted upon the recipient. It is possible that there could be a scenario where there is an increase, and then an increase, and then a decrease, and then another decrease, where the last decrease corresponds to the cognitive load that will be inflicted upon the recipient. As will be inferred from the aforementioned scenario, the magnitudes of the increases and the decreases can be different for each respective increase and/or decrease.

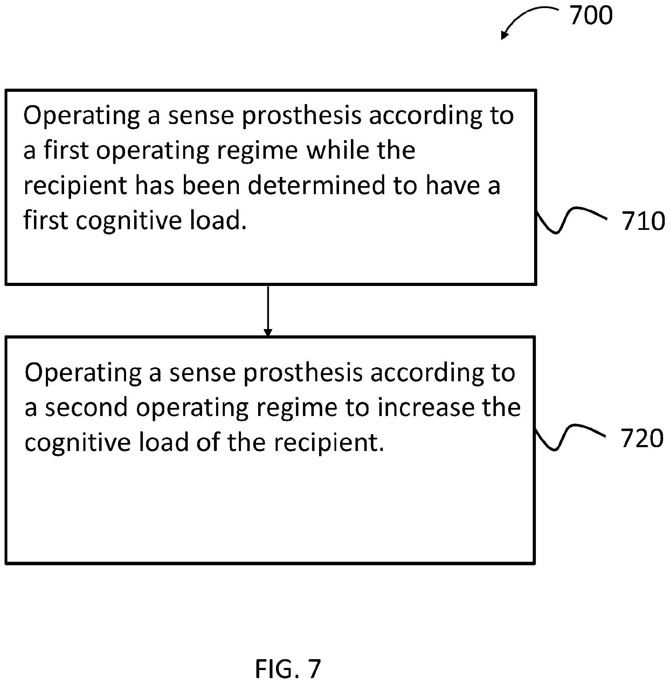

More specifically, now with reference to FIG. 7, there is an exemplary algorithm presented for an exemplary embodiment representing method 700. Method 700 includes method action 710, which entails operating a sense prosthesis according to a first operating regime while the recipient has been determined to have a first cognitive load. In an exemplary embodiment, the recipient has a lower cognitive load relative to that which was the case at a prior temporal period, as will be described below.

In an exemplary embodiment, the sense prosthesis is operated according to the second operating regime such that the stimulation rate of an electrode thereof is less than 600 pulses per second. In an exemplary embodiment, the hearing prosthesis is operated such that the electrode is stimulated at 500 pulses per second. In an exemplary embodiment, the sense prosthesis is operated such that the stimulation rate of an electrode is anywhere between 100 pulses per second and 1500 pulses per second or any value or range of values therebetween in one pulse per second increments. This can be considered a second scenario of use.

Method 700 further includes method action 720, which entails operating the sense prosthesis according to a second operating regime so as to increase the cognitive load relative to that which was the case during method action 710. Here, the recipient must devote more cognitive energy to understanding the sense evoked by the prosthesis relative to that which was the case during operation of the prostheses at the first operating regime. In an exemplary embodiment, the sense prosthesis is operated such that the stimulation rate of an electrode thereof is more than 600 pulses per second and less than 800 pulses per second. In an exemplary embodiment, the hearing prosthesis is operated such that the electrode is stimulated at 700 pulses per second. In an exemplary embodiment, the sense prosthesis is operated such that the stimulation rate of an electrode is anywhere between 400 pulses per second and 3000 pulses per second or any value or range of values therebetween in one pulse per second increments. This can be considered a first scenario of use, with a recipient has a low cognitive load.

At this time it is noted that in an exemplary embodiment, the varying of the aforementioned operating regimes can have utilitarian value with respect to increasing the cognitive load applied to the recipient vis-à-vis the evoked hearing percepts or sight percepts for a given amount of content extraction there from. Still further, the varying of the aforementioned operating regimes can have utilitarian value with respect to ensuring that the recipient is using the prosthesis to a more optimum value - a value that is commensurate with the recipient's ability at that time as opposed to a prior ability. With respect to utilizing different processing strategies, ACE vs. ACE with MP3 subscript 000 vs. some other processing strategy, the processing strategy that will be utilized will be the one that harder to relative to that which is the case for the reduced fatigue levels.

Some settings that could affect the level cognitive load associated in hearing (or seeing) with a specific map that is loaded into a prosthesis can include, by way of example, threshold and comfort levels; dynamic range; number and rate of stimulation per analysis frame; front-end signal processing algorithm settings (like AGCs); beam-former settings; signal Path Gains (like volume); etc. Accordingly, in an exemplary embodiment, one or more or all of the aforementioned settings can be adjusted or otherwise varied in real time or otherwise so as to manipulate or otherwise control the cognitive load inflicted upon the recipient.

In an exemplary embodiment, such as with respect to a hearing prosthesis, noise is inputted into the system so as to increase the cognitive load, at least in some instances. By way of example only and not by way of limitation, hearing situations where there is a lot of background noise increase the cognitive load. Accordingly, in some embodiments, the settings of the prosthesis may not necessarily be changed, but instead an additional signal is inputted into the sound that is captured or otherwise combined at the processing stage, so as to induce background noise that is not present. While the underlying concept is directed towards increasing the cognitive load, indirectly, this could be, in essence, like training the recipient to hear in noisy environments or otherwise familiarizing the recipient with a hearing in noisy environments. By way of example, a "cocktail-party" scenario can be one that is a challenge for the recipient of a hearing prosthesis. This can train the recipient to function in such a scenario.

In another exemplary embodiment, such as with respect to hearing prostheses, the sum of the features of sound that is captured is manipulated to make it actually harder to understand. By way of example only and not by way of limitation, the captured sound of someone speaking can be modified so that it has an accent (where none exists in reality). Still further by way of example, the frequency of the voice or sound can be changed. Indeed, such can have utilitarian value with respect to someone who is planning to go to, for example, France (or someone who is coming from France), where the speakers have a higher pitch to their voice. Any device, system, and/or method that can enable increased cognitive load while still enabling the recipient to function adequately with the hearing prosthesis can be utilized in at least some exemplary embodiments.

Of course, in some exemplary scenarios, the opposite of the above scenario where the cognitive load is increased is the case. For example, if a determination is made that the cognitive load is too high, even accounting for the fact that the operating regime has been set or otherwise is being used so as to increase the cognitive load, the operating regime can be changed so as to reduce the cognitive load. By rough analogy, this could be like downward adjusting the speed of a treadmill upon a determination that a runner's heart rate is higher than that which was desired.

It is noted that in at least some exemplary embodiments, the adjustments or otherwise settings that are used by the prostheses can also be "scene" dependent. For example, with respect to the hearing, in a hearing scene where there is a lot of background noise, it will thus be harder to hear. Accordingly, the cognitive load naturally increases. The devices, systems and/or methods take this into account. In this regard, the settings that are adjusted while taking in to account a given scene. By way of example only and not by way of limitation, in a scenario where a person is driving to work, the hearing prostheses can be set to have a cognitive load for the given sound environment, which would be relatively quiet with respect to a person driving a modern vehicle. Then, when the recipient stepped out of his or her car and left the parking garage to walk on a busy city street sidewalk, the hearing prosthesis is adjusted so that the cognitive load in the quiet sound environment is maintained or otherwise closely correlated. Accordingly, in an exemplary embodiment, the high cognitive load can be maintained, even though the adjustment settings during the second temporal period (while walking on the street) would otherwise result in a lower cognitive load if utilized during the first temporal period (while driving in the car).

Accordingly, in at least some exemplary embodiments, the management of cognitive load can be applied in real time or semi-real time. For example, the system could detect that the recipient is currently experiencing a high cognitive load - via connected sensory systems, forming part of a body area network. The system could then try altering settings to reduce the cognitive load on the recipient. In some exemplary embodiments, consistent with the embodiment detailed above, this alteration of the settings would reduce the cognitive load on the recipient so as to achieve the desired high cognitive load as opposed to simply reducing the cognitive load as a goal in and of itself.

Still, to be clear, some embodiments can be directed towards simply managing cognitive load to avoid overloading the recipient. In this regard, the devices, systems, and/or methods herein can be utilized in a cognitive load maintenance regime which maintains the cognitive load at a high level for habilitation and/or rehabilitation purposes, for example, and can also be utilized in a cognitive load management regime, which reduces the cognitive load in a scenario where a determination is made that the cognitive load has increased, without a goal or otherwise without constraints as to maintaining a high level of cognitive load. Indeed, with respect to the latter, in some instances, when in the cognitive load management regime, the system is utilized to reduce cognitive load to a relatively low level, in some and/or in all instances during a given temporal period. By way of example only and not by way of limitation, with respect to the cognitive load reduction regime, consider a scenario where the recipient is going to a bar or a party where he or she is trying to pick up, or otherwise simply trying to be seen as cool. The recipient is not going to want to have the cognitive load maintenance regime applied. Instead, the recipient is going to want to be in the most relaxed state possible. Accordingly, consider the example where the recipient is trying to have a conversation at a party. The system may detect that the recipient is currently expending a lot of effort to understand the person they are listening to, over the background noise, etc. The system then adjusts device settings, such as increase the beam-former directionality and/or the level and nature of the noise canceller, so as to reduce the cognitive load on the recipient in this situation. The aforementioned scenario which the teachings detailed herein enable can be utilized with respect to rehabilitation of a cochlear implant recipient. **In** this regard, in at least some exemplary embodiments, all of the recipients are recipients who previously had natural hearing otherwise had the capability of naturally hearing, and the cochlear implant is utilized to rehabilitate that hearing. Thus, in some exemplary embodiments, the aforementioned scenario (or any scenario detailed herein) occurs in the overall context of a program / hearing journey focused on rehabilitating a recipient's hearing, as opposed to simply trying to enable the recipient to have a hearing percept. Corollary to this is that in at least some exemplary embodiments, the recipient is utilizing his or her prosthesis as a cognitive load maintenance device, so as to improve the rate of rehabilitation (or habilitation) and then the recipient enters a scene where the recipient does not want to utilize the prosthesis as a cognitive load maintenance device, but instead as a cognitive load management device (e.g., a scene where the recipient is going to the party or the like). Thus, the prosthesis is "switched" to the cognitive load management regime during that period of time. The prosthesis is then later switched back to the cognitive load maintenance regime after the scene has changed. In at least some exemplary embodiments, the system is configured so as to gauge whether or not the prosthesis should be continued to be used as a cognitive load maintenance device when a scene changes. In an exemplary embodiment, intelligent learning can be implemented where the prosthesis or other part of the system "learns" what given scenes correlate to the recipient being likely to take the prosthesis out of the cognitive load maintenance regime. By way of example and not by way of limitation, the system can "remember" for what scenes the recipient has taken the device out of the cognitive load maintenance regime setting. This can be stored in the system, and when the system determines that the recipient is being exposed to a given scene where, in the past, the recipient at least once or sometimes or frequently takes the prosthesis out of the cognitive load maintenance regime, the prosthesis or system automatically takes the prosthesis out of the cognitive load maintenance regime.

Still further, in an exemplary embodiment, the prosthesis can be configured to evaluate and otherwise monitor the cognitive load of the recipient, and upon a determination that the recipient is experiencing too high of a cognitive load, the prostheses could automatically exit the cognitive load maintenance regime. By analogy, this would be like a pressure relief valve. The goal is to maintain pressure in a system, but to ensure that the pressure does not exceed a certain amount, and upon the occurrence thereof, it is possible that the system pressure will be lowered to a value lower than that which is desired.

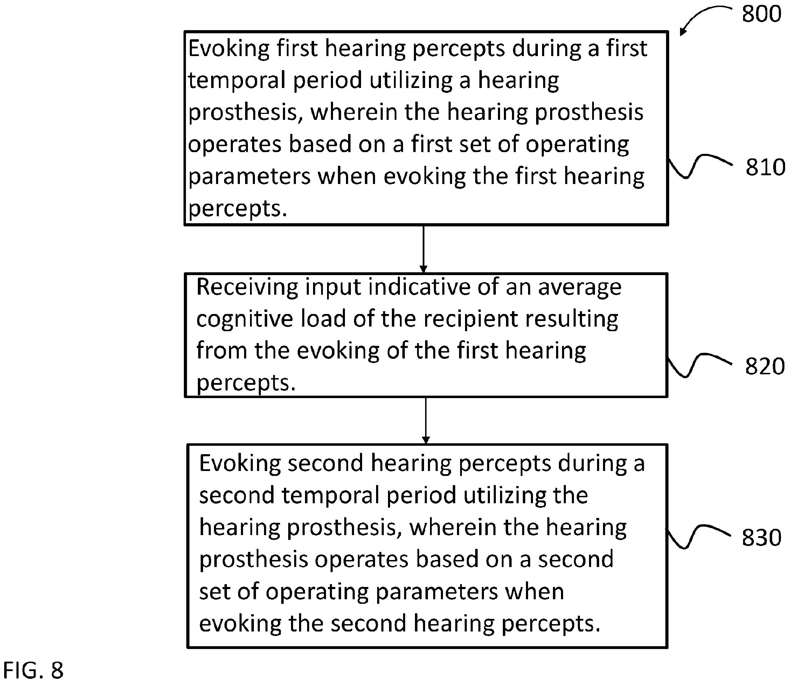

FIG. 8 presents an algorithm for another exemplary embodiment of a method, method 800. Method 800 includes method action 810, which includes evoking first hearing percepts during a first temporal period utilizing a hearing prosthesis, wherein the hearing prosthesis operates based on a first set of operating parameters when evoking the first hearing percepts. Method 800 also includes method action 820, which includes receiving input indicative of an average cognitive load of the recipient resulting from the evoking of the first hearing percepts. In an exemplary embodiment, the average cognitive load can be determined utilizing mean, median, and/or mode analysis over a temporal period of time utilizing multiple data points over the temporal period of time. Any device, system, and/or method that can enable the average cognitive load to be determined can be utilized in at least some exemplary embodiments. It is also noted that in at least some embodiments, an average cognitive load is not used. Instead, a non-statistically manipulated cognitive load data is utilized. Method 800 also includes method 830, which includes evoking second hearing percepts during a second temporal period (which can be a period after the first) utilizing the hearing prosthesis, wherein the hearing prosthesis operates based on a second set of operating parameters when evoking the second hearing percepts. In this embodiment, a switch from the first set of operating parameters to the second set of operating parameters is executed to increase the average cognitive load on the recipient that results from the evoking of the second hearing percepts.

In some embodiments of method 800, the method is part of a hearing rehabilitation method and/or not part of a hearing habilitation method. In some exemplary embodiments, the hearing rehabilitation method of which the method 800 is a part is executed so as to exercise the recipient's brain or otherwise maintain a level of cognitive load that challenges the recipient in a manner that improves the recipient's ability to hear relative to that which would be the case if method 800 was not executed. In an exemplary embodiment, the first temporal period lasts D days, weeks or months, and D is 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, 60, 90, 120, 150, 200, 250, 300, 350, 400, 500, or 600 or more, and the second temporal period lasts P days, weeks or months, where P is 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, 60, 90, 120, 150, 200, 250, 300, 350, 400, 500, or 600 or more.

It is noted that in an exemplary embodiment, method 800 includes additional actions which repeat method actions 820 and 830 one or two or three or four or five or six or seven or eight or nine or 10 or 11 or 12 or 13 or 14 or 15 or 16 times or more, except for the preceding average cognitive loads and for respective temporal periods after the preceding temporal period, where the respective temporal periods can individually have any of the values of D. thus, in an exemplary embodiment where method actions 820 and 830 are repeated 3 times after being first executed, method action 830 would have a third temporal period, a fourth temporal period, and a fifth temporal period, and a third set of operating parameters, and a fourth set of operating parameters in a fifth set of operating parameters, and so on.

Thus, in an exemplary embodiment of method action 810, the first temporal period lasts at least about two weeks. It is noted that in at least some exemplary embodiments, the first set of operating parameters can change during the first temporal period, and the second set of operating parameters can change during the second temporal period, and so on. In this regard, as noted above, in at least some exemplary embodiments, cognitive load can be reduced for certain circumstances (e.g., because the recipient is partying, etc.). Accordingly, in at least some exemplary embodiments, the second set of operating parameters, etc., are utilized to evoke a hearing percept for a percentage of time out of all of the hearing percept evoked during those temporal periods. For example, in an exemplary embodiment, the percentage of time that any given set of operating parameters is utilized out of a total time where hearing percepts are evoked for that temporal period corresponds to less than or more than or equal to at least about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, 60, 70, 80, 90, or 100% or any value or range of value therebetween in 0.1% increments.

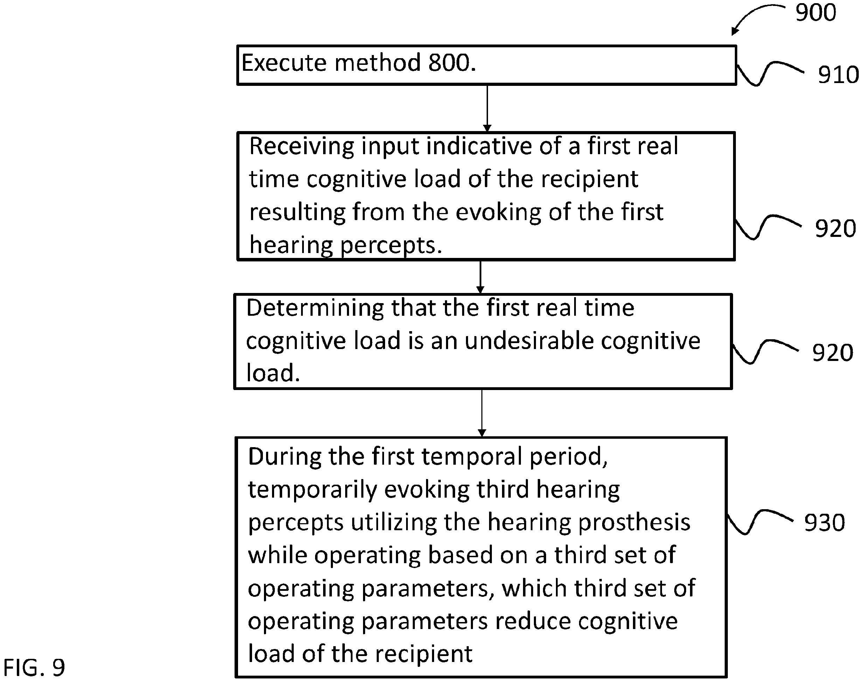

In view of the above, FIG. 9 presents an exemplary algorithm for another exemplary method, method 900. Method 900 includes method action 910, which includes executing method 800. Method 900 also includes method action 920, which includes receiving input indicative of a first real time cognitive load of the recipient resulting from the evoking of the first hearing percepts. In an exemplary embodiment, this can be achieved utilizing the sensors of the prostheses or the system of which this prosthesis is a part. In an exemplary embodiment, this can correspond to input from the recipient. Method 900 also includes method action 930, which includes determining that the first real time cognitive load is an undesirable cognitive load. In an exemplary embodiment, this can include a determination that the cognitive load is simply too high for the recipient, even beyond a level desired to exercise the recipient utilizing higher cognitive loads. Method 900 also includes method action 940, which includes during the first temporal period, temporarily evoking third hearing percepts utilizing the hearing prosthesis while operating based on a third set of operating parameters, which third set of operating parameters reduce cognitive load of the recipient.

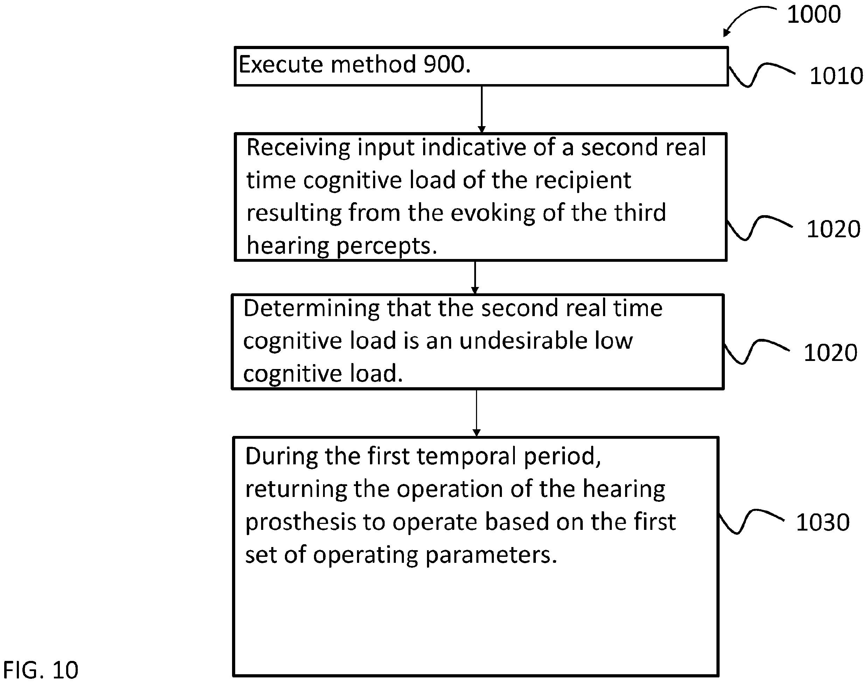

FIG. 10 depicts another exemplary algorithm for an exemplary method, method 1000. Method 1000 includes method action 1010, which includes executing method 900. Method 1000 also includes method action 1020, which includes receiving input indicative of a second real time cognitive load of the recipient resulting from the evoking of the third hearing percepts. Such can be executed according to any of the teachings detailed herein. Method 1000 also includes method action 1030, which includes determining that the second real time cognitive load is an undesirable low cognitive load. Method action 1030 is followed by method action 1040, which includes, during the first temporal period, returning the operation of the hearing prosthesis to operate based on the first set of operating parameters.

It is noted that in an exemplary embodiment, method action 920 and/or 1020 can be executed utilizing the supplemental assessment detailed above associated with figure 2. It is noted that this can be the case for any of the actions associated with follow-up evaluation or otherwise monitoring of cognitive load so as to implement the teachings detailed herein, in at least some exemplary embodiments. It is also noted that the system of figure 3 can be utilized to implement at least some of the method actions associated with methods 900 and/or 1000. Indeed, in some exemplary embodiments, the system depicted in figure 3 can be utilized to implement at least portions of any of the embodiments detailed herein and/or variations thereof, at least when the proper software and/or firmware and/or hardware is implemented therein.

In an exemplary embodiment of any of the methods 900 and 1000, in an exemplary embodiment where the hearing prosthesis is a cochlear implant, the first of operating parameters can be a first map of the cochlear implant, and the third set of operating parameters are not map-related parameters. In this regard, in an exemplary embodiment, the map need not be changed during the first temporal period, but instead, other parameters, such as for example, a beamforming parameter, a gain control, etc., are adjusted. In this regard, when the actions are executed so as to take into account in undesirably high cognitive load, the prosthesis can attempt to reduce the cognitive load by adjusting non-map related parameters, while maintaining the map for the cognitive load maintenance efforts. Thus, in an exemplary embodiment, any adjustments to the parameters or changes to the parameters due to an unacceptably high cognitive load can be non-map related parameters, and any adjustments to the parameters or changes to the parameters to increase the average cognitive load from a prior average cognitive load for the purposes of exercising the recipient, can be map related adjustments.

In view of the above, one way of looking at method 800 is to consider the temporal periods to be long-term training periods associated with a hearing journey, and the adjustments to the parameters or otherwise changes to the parameters are directed towards increasing the cognitive load for those long-term training periods. These periods can be considered habilitation or rehabilitation periods. These periods can be demarcated based on empirical evidence that the recipient has improved in his or her ability to use the prosthesis relative to the beginning of the prior period.

Thus, expanding on the above method, there is a method that further comprises determining that the cognitive load on the recipient that results from the evoking of the second hearing percepts is one of too low or too high, and switching from the second set of operating parameters to a third set of operating parameters and evoking third hearing percepts during a third temporal period after the first and second temporal periods, wherein the hearing prosthesis operates based on the third set of operating parameters when evoking the third hearing percepts, wherein a switch from the second set of operating parameters to the third set of operating parameters is executed to one of increase the cognitive load on the recipient that results from evoking of the third hearing percepts or decrease the cognitive load on the recipient that results from evoking of the third hearing percepts, respectively based on the determination that the cognitive load on the recipient that results from evoking of the second hearing percepts is one of too high or too low. In an exemplary embodiment, the action of determining that the cognitive load is one of too low or too high is a determination that it is too high, while in an alternate embodiment, the action of determining that the cognitive load is one of too low or too high is a determination that it is too low.

Further, in an exemplary embodiment expanding upon the method detailed above, there is a method that includes determining that the cognitive load on the recipient that results from the evoking of the third hearing percepts is one of too low or too high, and switching from the third set of operating parameters to a fourth set of operating parameters and evoking fourth hearing percepts during a fourth temporal period after the first and second and third temporal periods, wherein the hearing prosthesis operates based on the fourth set of operating parameters when evoking the fourth hearing percepts, wherein a switch from the third set of operating parameters to the fourth set of operating parameters is executed to one of increase the cognitive load on the recipient that results from evoking of the fourth hearing percepts or decrease the cognitive load on the recipient that results from evoking of the fourth hearing percepts, respectively based on the determination that the cognitive load on the recipient that results from evoking of the third hearing percepts is one of too high or too low. In an exemplary embodiment, the action of determining that the cognitive load is one of too low or too high is a determination that it is too high, while in an alternate embodiment, the action of determining that the cognitive load is one of too low or too high is a determination that it is too low.

Still further, expanding upon the above method, in an exemplary embodiment, there is a method that further includes the action of determining that the cognitive load on the recipient that results from the evoking of the fourth hearing percepts is one of too low or too high, and switching from the fourth set of operating parameters to a fifth set of operating parameters and evoking fifth hearing percepts during a fifth temporal period after the first and second and third and fourth temporal periods, wherein the hearing prosthesis operates based on the fifth set of operating parameters when evoking the fifth hearing percepts, wherein a switch from the fourth set of operating parameters to the fifth set of operating parameters is executed to one of increase the cognitive load on the recipient that results from evoking of the fifth hearing percepts or decrease the cognitive load on the recipient that results from evoking of the fifth hearing percepts, respectively based on the determination that the cognitive load on the recipient that results from evoking of the fourth hearing percepts is one of too high or too low. In an exemplary embodiment, the action of determining that the cognitive load is one of too low or too high is a determination that it is too high, while in an alternate embodiment, the action of determining that the cognitive load is one of too low or too high is a determination that it is too low.

Still further, expanding upon the above method, in an exemplary embodiment, there is a method that further includes, starting with N=1, the action of determining that the cognitive load on the recipient that results from the evoking of the N+4th hearing percepts is one of too low or too high, and switching from the N+4th set of operating parameters to a N+5th set of operating parameters and evoking N+6th hearing percepts during a N+5th temporal period after the first and second and third and fourth and N+4th temporal periods, wherein the hearing prosthesis operates based on the N+5th set of operating parameters when evoking the N+5th hearing percepts, wherein a switch from the N+4th set of operating parameters to the N+5th set of operating parameters is executed to one of increase the cognitive load on the recipient that results from evoking of the N+5th hearing percepts or decrease the cognitive load on the recipient that results from evoking of the N+5th hearing percepts, respectively based on the determination that the cognitive load on the recipient that results from evoking of the N+4th hearing percepts is one of too high or too low, and this is repeated for N equals 2, 3, 4, 5, 6, 7, 8, 9, 10, 11, 12, 13, 14, 15, 16, 17, 18, 19 or 20 or more, and in some embodiments, for any integer value of N less than 1000 (e.g., 100, 125, 94, 555, etc.). In an exemplary embodiment, the action of determining that the cognitive load is one of too low or too high is a determination that it is too high, while in an alternate embodiment, the action of determining that the cognitive load is one of too low or too high is a determination that it is too low.

In some exemplary embodiments, the hearing prosthesis is a cochlear implant, the first set of operating parameters is a first map of the cochlear implant, and the second set of operating parameters is a second map of the cochlear implant. Accordingly, in some exemplary embodiments, the aforementioned scenario is a progressive maps scenario.

Another exemplary embodiment includes a method where first hearing percepts are evoked during a first temporal period utilizing a hearing prosthesis, wherein the first temporal period is a period in which the recipient effectively habilitates or rehabilitates his/her hearing with the hearing prosthesis. In this method, the operating parameters of the hearing prosthesis are adjusted during the first temporal period to maintain, on average, a heightened cognitive load in the recipient of the hearing prosthesis resulting from use of the hearing prosthesis. In an exemplary embodiment, the average can be the average for the entire temporal period. In some exemplary embodiments of this method, the heightened cognitive load resulting from use of the hearing prosthesis is relative to that of a statistically significant temporal period prior to the first temporal period. Again, in an exemplary embodiment, the first temporal period can be a temporal period that began after a determination that the recipient has improved his or her ability to understand utilizing the prosthesis, and thus the settings and the like of the prosthesis are adjusted so as to increase the cognitive load relative to that which was the case during the prior temporal period.

In an exemplary embodiment, the method is part of a habilitation or rehabilitation journey of the recipient in which progressive maps are applied, the average cognitive load is monitored during the method, and, based on the monitored average cognitive load, the maps are progressed so as to increasingly heighten the average cognitive load applied to the recipient.

It is noted that all uses of the phrase average cognitive load herein can correspond to, in some embodiments, the long-term average cognitive loads for periods lasting according to D or P, etc., as detailed above.

It is noted that in some exemplary embodiments of the aforementioned method, again, the method is part of a habilitation or rehabilitation journey of the recipient, the average cognitive load is monitored during the method and operating parameters of the hearing prosthesis are adjusted, over the long run, to avoid statistically significant underutilization of the recipient's cognitive capacity. In an exemplary embodiment, the recipient cognitive load is monitored automatically using a biometric apparatus.

As noted above, embodiments are directed towards managing the cognitive load during changing sound scenes and/or light scenes. Thus, in an exemplary embodiment of the aforementioned method, the method further includes the action of determining that for a first sub-period of the first temporal period, the recipient is in a first sound scene. With reference to the above scenario where the recipient is in his or her car, the first sound scene could be a sound scene of relative quiet. The method further includes the action of determining that for a second sub-period of the first temporal period, the recipient is in a second sound scene that makes it substantially harder to hear than the first sound scene. This second sound scene could correspond to the recipient walking down a busy street in a city during rush hour. The method also includes the action of adjusting the operating parameters so that the average cognitive load during the first sub-period and the second sub-period is at least about the same and is at least about the same as the heightened cognitive load. That said, in another exemplary method, there is still the method of determining that for a first sub-period of the first temporal period, the recipient is in a first sound scene, and determining that for a second sub-period of the first temporal period, the recipient is in a second sound scene that makes it substantially harder to hear than the first sound scene. However, in this exemplary method, there is the action of adjusting the operating parameters so that the average cognitive load during the first sub-period is substantially higher than that of the second sub-period, wherein the average cognitive load during the first sub-period is at least about the same as the heightened cognitive load. In this regard, the second sound scene could be a sound scene in which the recipient is listening to a patient, where the recipient is a medical doctor. The recipient does not want to have a high cognitive load applied while the recipient is listening to a patient describing what could be a potentially life-threatening illness. Hence, the average cognitive load for that second sound scene is thus reduced relative to the average cognitive load for the first sound scene.

In view of the above, it can be seen how a hearing prosthesis can be utilized as a hearing rehabilitative exercise machine. In this regard, with reference to figure 6, in an exemplary embodiment, there is a system, comprising a hearing prosthesis suite, such as that established by at least elements 410 and 420 detailed above. There is also in this system, a data input suite, such as, for example, that established by elements 431 and 430 detailed above. In this exemplary embodiment, the data input suite is configured to receive data indicative of a cognitive load of the recipient. It is noted that the aforementioned suites can include additional components or fewer components. In an exemplary embodiment, the system is configured to operate in a hearing rehabilitative exercise machine mode in which the system automatically adjusts operation of the hearing prosthesis based on data obtained by the data input suite to exercise the recipient, thereby rehabilitating the recipient. In this regard, the system can be a normal hearing prosthesis adapted to vary the cognitive load according to the regimes detailed herein so as to rehabilitate the recipient. It is noted that the system can be a part of a multi-component system, such as the system 600 shown in figure 6. It is also noted that the system can include remote components in signal communication with the hearing prosthesis suite, such as a smart phone or a smartwatch or a personal electronics system, etc., which can provide input to the system.

Consistent with the embodiment of figure 6, the data input suite can include a biometric suite, and the system can be configured to automatically adjust operation of the hearing prosthesis when in the exercise mode based on data obtained by the biometric suite to exercise the recipient, thereby rehabilitating the recipient. In some embodiments, the hearing prosthesis suite is a cochlear implant.

It is noted that while some embodiments can be a system with multiple components that are spatially separate from one another and are not connected to one another in a structural manner, in some other embodiments, the system is a unitary device, or at least portions thereof are such. For example, the hearing prosthesis suite and the input suite could be all part of a cochlear implant that includes the input suite.

**In** some embodiments, the hearing prosthesis suite is configured to, when in the exercise mode, automatically adjust operation of the hearing prosthesis based on a rehabilitation program that works in relationship with the data obtained by the data input suite to exercise the recipient, thereby rehabilitating the recipient. For example, if the rehabilitation program "requires" or otherwise should have recipient having a cognitive load of a first value, the system can receive input indicative of the recipients cognitive load, such as from the aforementioned biometric sensors of the like, evaluate that input, and then adjust the operating parameters or the like to maintain that first value. Also, in some embodiments, the system is configured to, when in the exercise mode, automatically adjust operation of the hearing prosthesis based on the data obtained by the data input suite to increase the cognitive load on the recipient. This is consistent with the teachings detailed above, where the goal of at least some embodiments is to maintain a heightened cognitive load. Conversely, in some embodiments, the system is configured to operate in a non-exercise mode. In some embodiments, the non-exercise mode does not react at all to cognitive load. In some embodiments, the non-exercise mode can react to cognitive load, but only to control the prosthesis to reduce cognitive load in a scenario where the cognitive load has become heightened. In some embodiments, the non-exercise mode can have multi-sub modes, such as respective sub modes corresponding to the two aforementioned non-exercise modes. In an exemplary embodiment of the non-exercise mode where the mode controls the prosthesis only to reduce cognitive load in a scenario where the cognitive load has become heightened, such reduces the cognitive load to a level below that which would be the case during the exercise mode (in at least some exemplary embodiments).

Accordingly, in some exemplary embodiments, the system is configured to, when out of the exercise mode, automatically adjust operation of the hearing prosthesis based on the data obtained by the data input suite to decrease the cognitive load on the recipient below a level corresponding to that which would exist when in the exercise mode and below a current level determined based on the data obtained by the data input suite. Also, in some exemplary embodiments, the system is configured to, when in the exercise mode, automatically adjust the operation to set the cognitive load on the recipient at a level higher than that which would be the case when the prosthesis is in a non-exercise mode where the prosthesis still adjust operation based on the cognitive load.

In an exemplary embodiment, the system is configured to, when in the exercise mode, automatically adjust operation of the hearing prosthesis based on the data obtained by the data input suite to increase the cognitive load on the recipient upon a determination that the recipient's current cognitive load is below a predetermined exercise level set for rehabilitation. In this regard, the predetermined exercise level set for rehabilitation can be the level set by a clinician or the like or according to an algorithm that improves or otherwise exercises the recipient. This is distinguished from a scenario where the cognitive load is simply increased because a prior hearing scene resulted in a cognitive load that was determined to be too much for the recipient and the cognitive load was reduced so as to improve hearing.

In some embodiments, the system is configured to log data indicative of the adjustment of operation of the hearing prosthesis. The hearing prosthesis itself can log the data, or a component remote from the hearing prosthesis, such as a smart phone or a portable electronics device can log the data. This data can be used, in some embodiments, in methods that evaluate the data so as to adjust the exercise regime. In an exemplary embodiment, the data that is log can be downloaded or uploaded to a device, such as the fitting system 370 or a remote device such as a personal computer or a server located in another geographic location entirely, with the data can be evaluated so as to determine if and/or how to adjust the exercise regime. In some embodiments, the system is configured to, when in the exercise mode, automatically adjust operation of the hearing prosthesis based on the data obtained by the data input suite and based on historical data to vary the cognitive load on the recipient thereby rehabilitating the recipient. This historical data can be the logged data. This historical data can be data developed based on the log data. The historical data can correspond to the prior average cognitive load or the like for a given temporal period, and the system can utilize the current data to maintain a cognitive load at a desired (predetermined, calculated, etc.) value above the historical average.

Embodiments are also directed to fitting a sense prosthesis. In some fitting session, recipients are fitted in a quiet and comfortable environment. The map parameters being fitted are thus likely to be appropriate for these type of environments. However, these settings will not represent the full picture / will likely, at least in some instances, undershoot the recipient's cognitive load that will result during normal use as the recipient has not been evaluated on how the recipient performs when under stress and/or simply having a high cognitive load burden. Thus, some of the teachings herein can be used to more accurately evaluate the actual cognitive load that will exist. In some instances, an induced/simulated environment (could be via virtual or mixed reality) could be utilized.

To be clear, the cognitive load has never been a parameter that has been considered during fitting. Thus, in some embodiments, there is a method of fitting that includes measuring and/or monitoring the cognitive load during a fitting session such that cognitive load can be accounted for in fitting. In some embodiments, this can allow the clinician to fit a map that is effective in terms of its cognitive load, or at least in part in terms of its cognitive load. In at least some embodiments, if two maps are able to achieve similar hearing outcomes, but one of them requires significantly less cognitive load for speech understanding, that map can be used in at least some embodiments. Indeed, in view of the teachings herein, one would presume that that would be the map to use. However, also in view of the teachings herein, in the case of rehabilitation (or habilitation), a map that is recording a lower cognitive load may indicate that insufficient sensorial stimulation is being presented to the recipient, and a better map could be determined or otherwise identified, again consistent with the teachings detailed herein.

While some embodiments herein are directed towards a scenario where in a given fitting session, a series of maps or the like are created, and a progressive maps regime is utilized to maintain a cognitive load, or otherwise the maps are correlated to the cognitive load. That said, in some exemplary embodiments, based on the cognitive load, a determination can be made as to whether or not to refit the prosthesis. For example, upon a determination that the average cognitive load of the recipient has decreased by certain amounts, or otherwise is not at the heightened level that is desired, a determination can be made to refit the recipient. Thus, in an exemplary embodiment, the fitting system 370 can be re-utilized at that time to develop a new map or otherwise develop new fitting settings to be implemented to heighten the average cognitive load relative to the existing map. That is, this could be a variation of the progressive maps concept where the new map is not predetermined, but instead developed at the time that it is determined that the old map is not sufficiently maintaining the cognitive load at a level that is desired. In some embodiments, the hearing prosthesis can be reprogrammed upon a determination that the average cognitive load is not that which is desired or otherwise utilitarian vis-à-vis implementing the teachings detailed herein.

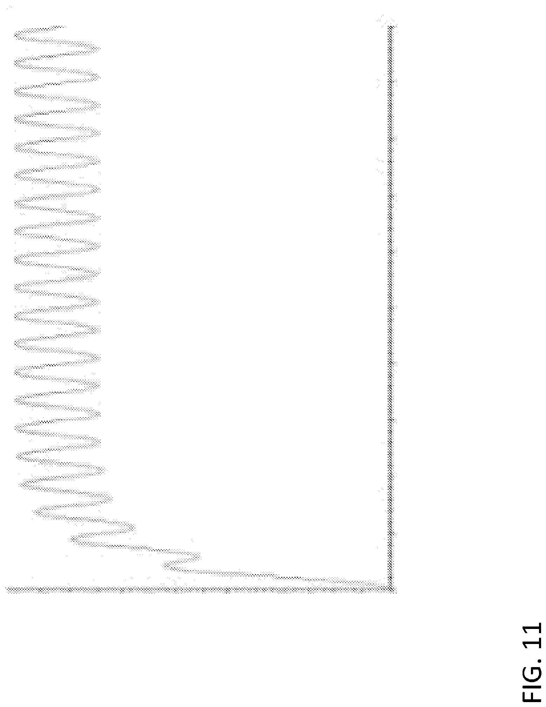

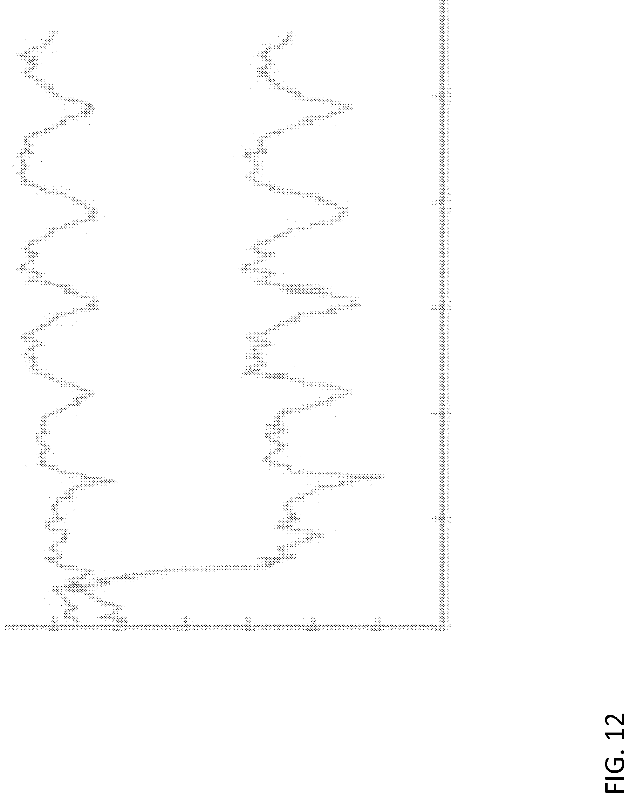

FIGs. 11 and 12 depict charts associated with a fitting session where stress / the environment is such that cognitive load is lower and where stress/the environment is such that cognitive load is higher, respectively. The x-axis is temporal, and the y-axis is a magnitude of the biometric parameter that is being monitored. The exact same sounds for threshold and comfort level are given to the recipient during the test. Using the calm response (figure 11) as a reference, the erratic response (figure 12) would indicate that the cognitive load of the individual is at a minimum, not stable, and likely higher. In an exemplary embodiment, during the fitting session, efforts are made to evaluate how the settings can be set to reduce the load burden in the stressed environment. By way of example only and not by way of limitation, in an exemplary embodiment, the number of maxima can be updated or otherwise changed (lowered in some embodiments). The stimulation rate can be changed (lowered in some embodiments). The threshold and/or comfort level for at least some of the electrodes and/or for at least some frequencies can be adjusted relative to that which is the case for the calm/unstressed environment.

The fitting session will be executed with a goal of finding a setting or settings in which the recipient, while under stress, will have less cognitive load applied to understand the sound environment around him or her, such as understanding speech, all other things being equal.

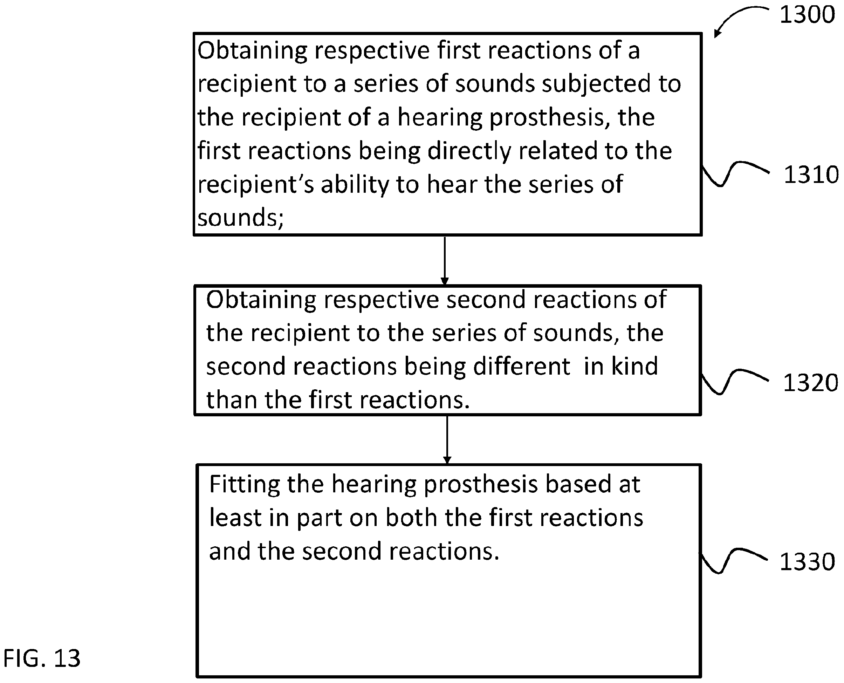

Thus, FIG. 13 presents an exemplary algorithm for an exemplary method, method 1300. Method 1300 includes method action 1310, which includes obtaining respective first reactions of a recipient to a series of sounds subjected to the recipient of a hearing prosthesis, the first reactions being directly related to the recipient's ability to hear the series of sounds. In some embodiments, this can correspond to the customary threshold and comfort level tests applied during fitting of a prosthesis, such as a cochlear implant. The reactions can correspond to the typical reactions of whether or not the recipient can hear anything, and whether or not what is heard is comfortable. Method 1300 further includes method action 1320, which includes obtaining respective second reactions of the recipient to the series of sounds, the second reactions being different in kind than the first reactions. Here, the second reactions can be the biometric parameters detailed above. Method 1300 further includes method action 1330, which includes fitting the hearing prosthesis based at least in part on both the first reactions and the second reactions. Thus, consistent with the teachings above, in an exemplary embodiment, the second reactions are reactions indicative of a cognitive load of the recipient.

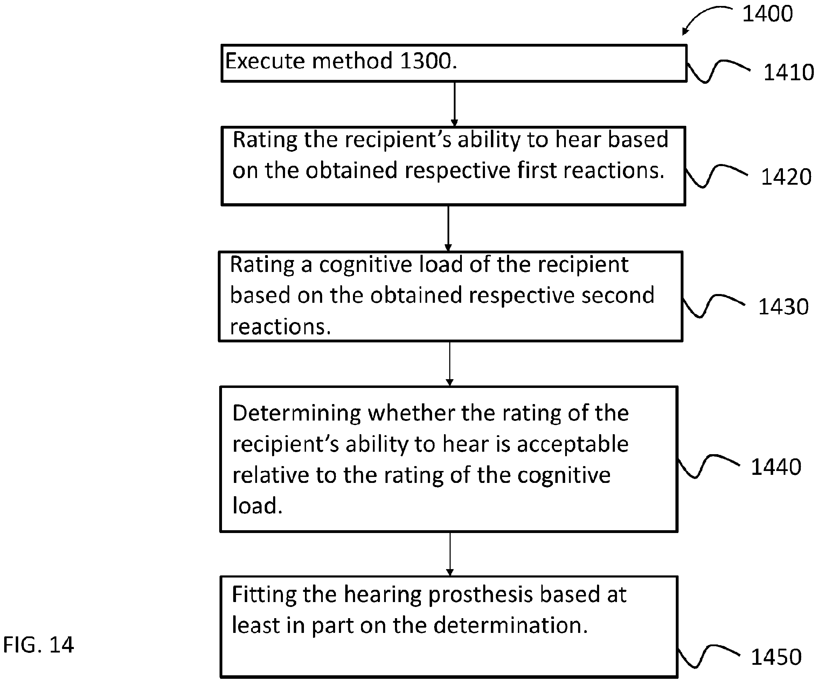

Figure 14 depicts another exemplary algorithm for an exemplary method, method 1400. Method 1400 includes method action 1410, which entails executing method 1300. Method 1400 also includes method action 1420, which includes rating the recipient's ability to hear based on the obtained respective first reactions. It is noted at this time that it is possible to execute method 1420 prior to the completion of method 1300. In this regard, it is noted that unless otherwise specified or unless the art does not enable such, any method action detailed herein can be practiced in any order relative to any other method action. Method 1400 also includes method action 1430, which includes rating a cognitive load of the recipient based on the obtained respective second reactions. Any method of rating can be executed. The simplest would be just declaring one to have an unacceptable or an acceptable cognitive load. Another way would be to quantify the cognitive load in some manner. This also the case with respect to the rating of the recipient's ability to hear. Any way of rating the cognitive load and/or the recipient's ability to hear can be utilized in at least some exemplary embodiments.

Method 1400 further includes method action 1440, which includes determining whether the rating of the recipient's ability to hear is acceptable relative to the rating of the cognitive load. By way of example only, in an exemplary scenario, the ability to hear could be rated as stellar, but the cognitive load can be rated as torture. Hence, this might be deemed less than utilitarian. Conversely, the ability to hear could be rated as excellent, and the cognitive load can be rated as low. Hence, this might be deemed very utilitarian. That said, in an environment where the goal is to rehabilitate or debilitate the recipient, that might be deemed to be less desirable than, for example, a rating where the ability to hear is excellent, but the cognitive load is rated as medium. In at least some exemplary scenarios, that one might be chosen over the other one. Method 1400 further includes method action 1450, which includes fitting the hearing prosthesis based at least in part on the determination. Note that method action 1450 can be executed simultaneously or as part of method action 1330. Indeed, in at least some exemplary embodiments, method action 1450 is simply an extended version of method action 1330.

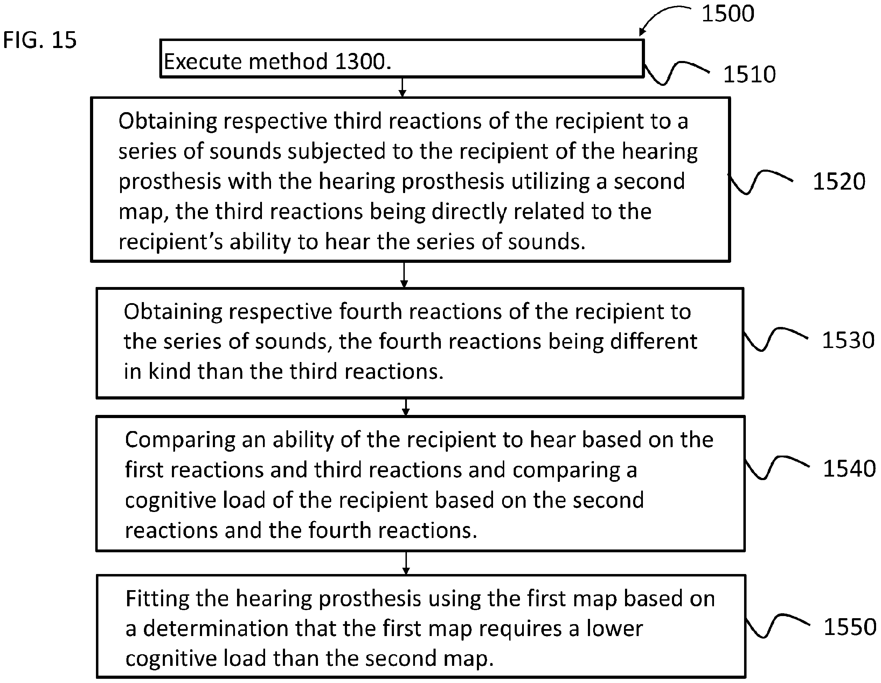

In some embodiments of the fitting methods detailed above, the obtained respective first reactions and the obtained respective second are obtained with the hearing prosthesis utilizing a first map. In this regard, figure 15 presents an exemplary algorithm where method 1300 is executed in method 1500 utilizing a first map. Method 1500 includes method action 1510, which includes executing method 1300 for that first map. Method 1500 also includes method action 1520, which includes obtaining respective third reactions of the recipient to a series of sounds subjected to the recipient of the hearing prosthesis with the hearing prosthesis utilizing a second map, the third reactions being directly related to the recipient's ability to hear the series of sounds. Method 1500 also includes method action 1530, which includes, obtaining respective fourth reactions of the recipient to the series of sounds, the fourth reactions being different in kind than the third reactions (again, this can be a reaction associated with cognitive load). Method 1500 also includes method action 1540, which includes comparing an ability of the recipient to hear based on the first reactions and third reactions and comparing a cognitive load of the recipient based on the second reactions and the fourth reactions. Method 1500 includes with method action 1550, which again can be part of method 1330, which includes fitting the hearing prosthesis using the first map based on a determination that the first map requires a lower cognitive load than the second map. To be clear, it is noted that method actions 1520-1540 can be executed before method actions 1310 and 1320, consistent with the note above indicating that the method actions can be executed in any order or that can enable the teachings herein. In this regard, it is to be noted that the nomenclature "first," "second," etc., are utilized simply for nomenclature purposes, and do not connote in all instances temporal or primacy information (unless otherwise indicated).

In an exemplary embodiment of method 1500, there is also the action of determining that the ability of the recipient to hear using the first map includes determining that the recipient can hear using the first map at least about the same or better than the ability of the recipient to hear using the second map. Thus, the first map would be chosen in an embodiment where the goal is to reduce the cognitive load, all other things being equal. That said, consistent with the teachings above, in some embodiments, the map that requires more cognitive load might be chosen. (Note that in some embodiments, the map that requires more cognitive load might be saved for later date such that the progressive maps scenario can be implemented (more on this below)).

Thus, in an exemplary embodiment, there is a variation of method 1500, where method actions 1510-1540 are executed as detailed above. However, method action 1550 is replaced with a slightly different action, which includes fitting the hearing prosthesis using the first map based on a determination that the first map requires a higher cognitive load than the second map. In an exemplary implementation of this variation of method 1500, the action of determining that the ability of the recipient to hear using the first map includes determining that the recipient can hear at least about the same or better than the ability of the recipient to hear using the second map. That said, in an alternate embodiment, the action of determining that the ability of the recipient to hear using the first map could be determining that the recipient does not hear quite as good as that which is the case with respect to the second map. In such an embodiment, the desire to have an increased cognitive load might override the utility of hearing, as counterintuitive as that may seem to the person of ordinary skill in the art.

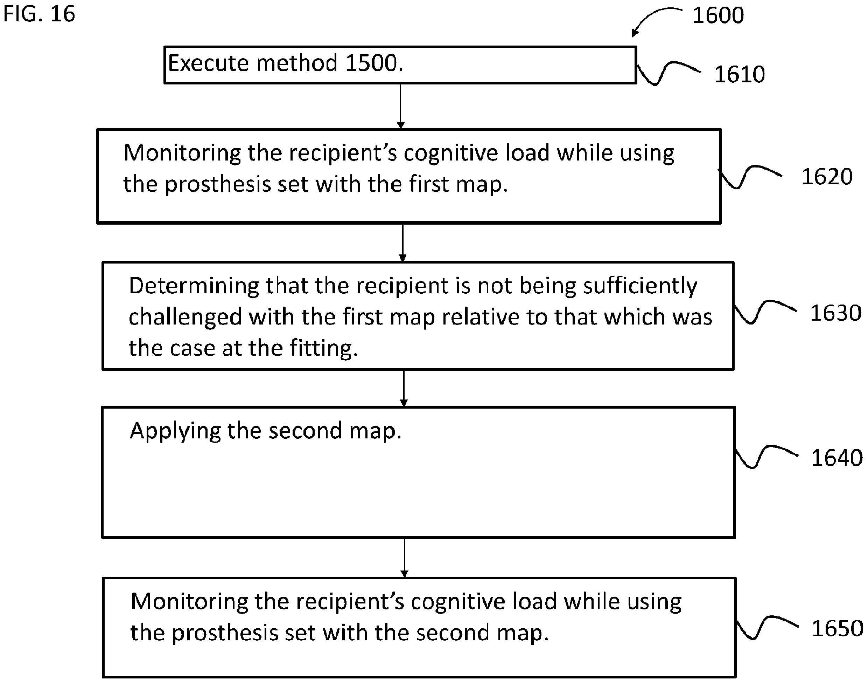

As noted above, in some embodiments, the map that requires more cognitive load might be saved for later date. Accordingly, in an exemplary embodiment, there is method 1600 is represented in the algorithm in figure 16. Here, method 1600 includes method action 1610, which includes executing method 1500. In this regard, the second map is saved, but not used to fit the prostheses in method action 1550. The recipient then goes out to the world and utilizes his or her prostheses, meanwhile, at method action 1620, the recipient's cognitive load is monitored while the recipient utilizes the prosthesis set with the first map. This can be done according to any of the teachings detailed herein, over a period of time of D or P, etc. The action of monitoring the recipient's cognitive load does not require an evaluation of the cognitive load. Instead, in an exemplary embodiment, it is the collection of the biometric sensor data, for example. Consistent with the embodiment detailed above, the prosthesis or otherwise the system can log this data. That said, in an exemplary embodiment, the action of monitoring the recipient's cognitive load can be executed by having the recipient indicate in some form or another how taxed he or she is on at a day to day or week to week or month to month basis, or whatever basis, etc. Indeed, in an exemplary embodiment, such can entail simply an evaluation of how many times the recipient downgrades or otherwise change is a setting so as to reduce the cognitive load. If a period of time in the case of the recipient is not downgrading the performance of the prosthesis to reduce the cognitive load applied thereby, this can indicate that the recipient is not being subjected to heightened cognitive loads.

Any device, system, and/or method that will enable the monitoring of the recipients cognitive load can be utilized in some embodiments.

Method 1600 also includes method action 1630, which includes determining that the recipient is not being sufficiently challenged with the first map relative to that which was the case at the fitting or relative to any other data point that has utilitarian value. In this regard, the data logged during method action 1620 can be evaluated, and this determination can be made. Method 1600 includes method action 1640, which includes applying the second map. Again, this can be executed at the end of the temporal period lasting D or P days, etc. Method 1600 also includes method action 1650, which includes monitoring the recipient's cognitive load while using the prosthesis set with the second map. This can correspond to basically going back to method action 1620, except for the second map. This process can be repeated a number of times for additional maps etc. so as to provide progressive maps. In an exemplary embodiment, after the second map is applied, and/or after the second map is utilized in conjunction with the monitoring for a given period of time, and/or after a determination is made that the recipient is not being sufficiently challenged with the second map, or after any other caveat that can have utilitarian value, etc., the recipient can be subjected to additional tests alike in a new fitting session, where, for example, method 1500 would be executed for a third and/or a fourth and/or a fifth map, etc., where those maps can be utilized to continue the progressive maps effort.

As noted above, in an exemplary embodiment, the fitting methods can be executed while subjecting the recipient to an environment where a stimulus that will increase the cognitive load, such as that which will increase the stress of the recipient. In an exemplary embodiment, the actions of fitting can be executed with the background of a baby crying, or a noisy city street, or for example listening to a speech given by a politician to which the recipient is adverse (e.g., a Trump speech for a Clinton supporter, and *vice versa*)*.* In an exemplary embodiment, the actions of fitting can be executed while inserting background noise or any other stimulus that will make listening harder. Accordingly, in an exemplary embodiment, there is a method of executing a fitting operation for the hearing prostheses where the fitting operation includes stimulus that will increase the cognitive load required for the recipient to hear. In an exemplary embodiment, this can result in a more utilitarian map for the recipient as it is developed in a scenario for a more real world or for a more difficult hearing scenario relative to that which would be the case in the absence of the stimulus. In an exemplary embodiment, this method of fitting further includes developing a map that reduces the cognitive load in the face of the stimulus. Again, as noted above, for example, the various thresholds and/or comfort levels can be set differently so as to help the recipient cope with this higher cognitive load environment. Indeed, in an exemplary embodiment, a map can be developed especially for stress situations, etc., and can be applied automatically or at the request of the recipient upon a determination that the recipient is subjected to stress.

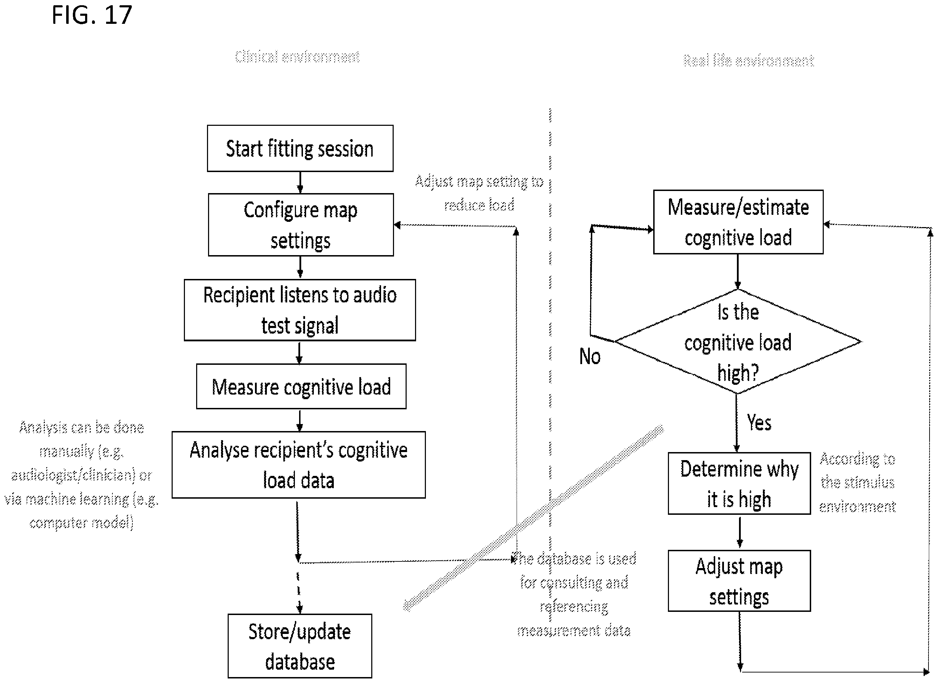

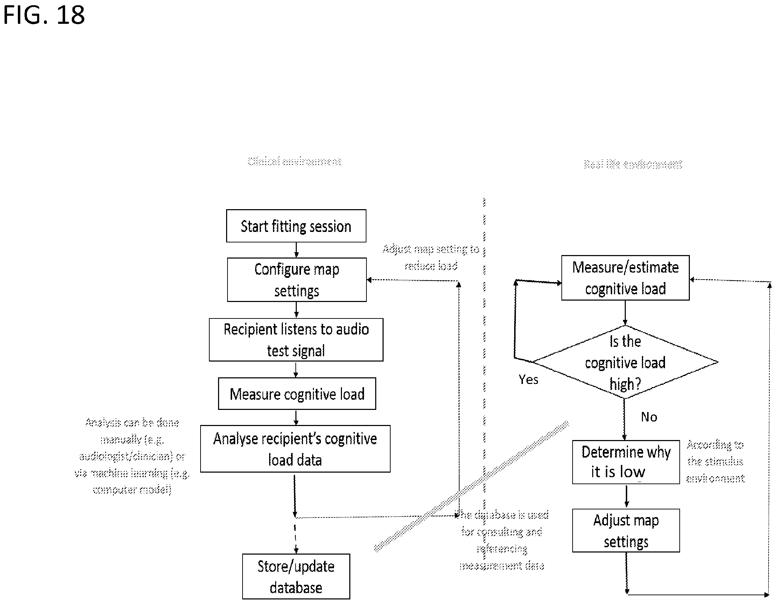

FIGs. 17 and 18 present some exemplary flowcharts for some exemplary methods. In both of the figures, the chart on the left side is the same, and is related to a fitting session. The chart on the right side represents two scenarios with respect to handling cognitive load. Figure 17 represents the scenario where the goal is to prevent the cognitive load from being too high, and thus the map settings (as opposed to the general operating parameters, such as implementation of beamforming, or noise cancellation) are adjusted to lower the cognitive load. Conversely, figure 18 represents a scenario where the goal is to prevent the cognitive load from being too low, and thus the map settings are adjusted to increase the cognitive load. As can be seen, these embodiments also represent the logging of data as represented from the arrow crossing from the real-life environment to the clinical environment. This is a representative / conceptual concept. In an exemplary embodiment, the log data could first be stored on the real-life environment side, such as in the memory of the smart phone or other component utilized with the prosthesis, or in the prosthesis itself, and then transferred to the database.

It is briefly noted that in at least some exemplary embodiments of executing the methods represented by figures 17 and 18, time averages or other statistical analysis can be applied to the results of the determination of the cognitive load. In this regard, it could be that there are extraneous data points that create abnormally high cognitive load and/or abnormally low cognitive load situations, which will skew the data or the like. Accordingly, by time averaging the data or by performing statistical analysis (e.g., such as, for example, removing the data points that are a standard deviation from what otherwise would be the average, applying a least mean squares analysis, etc.), more accurate, or at least significant, data can be obtained and then the ultimate determination of whether or not the cognitive load is high can be made (or low, note that in some embodiments, the methods of the flowcharts of 17 and 18 are executed based on a determination that the cognitive load is low instead of high).

With respect to the figures flowcharts in figures 17 and 18, in an exemplary embodiment, after the evaluation of whether or not the cognitive load is high, and analysis of why that is the case can be executed, and if an extraneous factor or the like is determined to be present, the results of that determination can be weighted or otherwise discounted. By way of example only and not by way of limitation, in a scenario where there is a determination that the cognitive load is high, and evaluation can be performed as to why that is the case. It could be that the reason that the cognitive load is high is unrelated to anything associated or at least is unrelated in a significant matter to anything associated with the hearing prosthesis. For example, the recipient can be going through a traumatic experience in his or her life, or could be overly fatigued in general, or could be suffering from some element or the like that would otherwise skew the data. Accordingly, in an exemplary embodiment, the action of determining whether or not the cognitive load is high and/or the action of determining what to do there about is also influenced by correlating such to other factors which could have an influence there on.

It is further noted that any disclosure of a device and/or system detailed herein also corresponds to a disclosure of otherwise providing that device and/or system.

Any disclosure of any method action herein corresponds to a disclosure of a device and/or system configured to implement that method action or otherwise having that functionality. In an exemplary embodiment, such can be achieved via the programming of a processor or microprocessor or other computer chip having input output and control functions and otherwise utilizing an electronic circuit having logic circuits and configured to receive input and output such as by wired terminals, which input and output can be electrical signals, which signals can be provided to the circuitry and to the logic and/or to the processor/microprocessor, etc., which can be programmed or otherwise configured via solid-state electronics or the use of firmware, etc., to have such functionality. Any disclosure of any device and/or system having functionality corresponds to a method that implements that functionality. Any disclosure of any action of manufacturing or otherwise creating a device and/or system corresponds to a device and/or system that results from such actions. Any disclosure of any device and/or system herein also corresponds to a disclosure of making or otherwise providing such device and/or system.

While various embodiments have been described above, it should be understood that they have been presented by way of example only, and not limitation. It will be apparent to persons skilled in the relevant art that various changes in form and detail can be made therein without departing from the scope of the present invention which is defined by the appended claims.

## Claims

1. A system, comprising:
a hearing prosthesis suite; and
a data input suite configured to receive data indicative of a cognitive load of a recipient, wherein
the system is configured to operate in a hearing rehabilitative exercise machine mode in which the system automatically adjusts operation of the hearing prosthesis based on data obtained by the data input suite to exercise the recipient, thereby rehabilitating the recipient,
the data input suite includes a biometric suite; and
the system is configured to automatically adjust operation of the hearing prosthesis when in the exercise mode based on biometric data of the recipient obtained by the biometric suite to exercise the recipient, thereby rehabilitating the recipient,
wherein the biometric suite includes a module that is configured to analyze biometric signals recorded from sensors in real-time and use this information to form a mental state of the recipient with respect to his/her present cognitive load.

2. The system of claim 1, wherein:
the hearing prosthesis suite is configured to, when in the exercise mode, automatically adjust operation of the hearing prosthesis based on a rehabilitation program that works in relationship with the data obtained by the data input suite to exercise the recipient, thereby rehabilitating the recipient.

3. The system of claim 1, wherein:
the system is configured to, when in the exercise mode, automatically adjust operation of the hearing prosthesis based on the data obtained by the data input suite to increase the cognitive load on the recipient upon a determination that the recipient's current cognitive load is below a predetermined exercise level set for rehabilitation, or
the system configured to, when in the exercise mode, automatically adjust operation of the hearing prosthesis based on the data obtained by the data input suite and based on historical data to vary the cognitive load on the recipient thereby rehabilitating the recipient.

4. The system of claim 1, wherein:
the system configured to log data indicative of the adjustment of operation of the hearing prosthesis.

5. The system of one of the claims 1-4, wherein the system is configured for:
obtaining respective first reactions of a recipient to a series of sounds subjected to the recipient of a hearing prosthesis, the first reactions being directly related to the recipient's ability to hear the series of sounds;
obtaining respective second reactions of the recipient to the series of sounds, the second reactions being different in kind than the first reactions; and
fitting a hearing prosthesis based at least in part on both the first reactions and the second reactions.

6. The system of claim 5, wherein:
the second reactions are reactions indicative of a cognitive load of the recipient.

7. The system of claim 6, wherein the system is further configured for:
rating the recipient's ability to hear based on the obtained respective first reactions; rating a cognitive load of the recipient based on the obtained respective second reactions;
determining whether the rating of the recipient's ability to hear is acceptable relative to the rating of the cognitive load;
fitting the hearing prosthesis based at least in part on the determination.

8. The system of claim 5, wherein:
the obtained respective first reactions and the obtained respective second reactions are obtained with the hearing prosthesis utilizing a first map;
the system is further configured for:
obtaining respective third reactions of the recipient to a series of sounds subjected to the recipient of the hearing prosthesis with the hearing prosthesis utilizing a second map, the third reactions being directly related to the recipient's ability to hear the series of sounds;
obtaining respective fourth reactions of the recipient to the series of sounds, the fourth reactions being different in kind than the third reactions; comparing an ability of the recipient to hear based on the first reactions and third reactions and comparing a cognitive load of the recipient based on the second reactions and the fourth reactions; and
fitting the hearing prosthesis using the first map based on a determination that the first map requires a lower cognitive load than the second map.

9. The system of claim 5, wherein:
the obtained respective first reactions and the obtained respective second reactions are obtained with the hearing prosthesis utilizing a first map;
the system is further configured for:
obtaining respective third reactions of the recipient to a series of sounds subjected to the recipient of the hearing prosthesis with the hearing prosthesis utilizing a second map, the third reactions being directly related to the recipient's ability to hear the series of sounds;
obtaining respective fourth reactions of the recipient to the series of sounds, the fourth reactions being different in kind than the third reactions;
comparing an ability of the recipient to hear based on the first reactions and third reactions and comparing a cognitive load of the recipient based on the second reactions and the fourth reactions; and
fitting the hearing prosthesis using the first map based on a determination that the first map requires a higher cognitive load than the second map.

10. The system of claim 9, wherein the system is further configured for:
determining that the ability of the recipient to hear using the first map includes determining that the recipient can hear at least about the same or better than the ability of the recipient to hear using the second map.

11. The system of claim 1, wherein the system is further configured for:
evoking first hearing percepts during a first temporal period utilizing a hearing prosthesis, wherein the hearing prosthesis operates based on a first set of operating parameters when evoking the first hearing percepts;
receiving input indicative of an average cognitive load of the recipient resulting from the evoking of the first hearing percepts; and
evoking second hearing percepts during a second temporal period utilizing the hearing prosthesis, wherein the hearing prosthesis operates based on a second set of operating parameters when evoking the second hearing percepts,
wherein
a switch from the first set of operating parameters to the second set of operating parameters is executed to increase the average cognitive load on the recipient that results from the evoking of the second hearing percepts.

12. The system of claim 11, wherein:
the hearing prosthesis is a cochlear implant;
the first set of operating parameters is a first map of the cochlear implant;
and the second set of operating parameters is a second map of the cochlear implant.

13. The system of one of the claims 1 - 12, wherein:
recipient cognitive load is monitored automatically using the biometric suite.

14. The system of claim 11, wherein the system is further configured for:
determining that for a first sub-period of the first temporal period, the recipient is in a first sound scene;
determining that for a second sub-period of the first temporal period, the recipient is in a second sound scene that makes it substantially harder to hear than the first sound scene;
adjusting the operating parameters so that the average cognitive load during the first sub-period and the second sub-period is at least about the same and is at least about the same as the heightened cognitive load, or
adjusting the operating parameters so that the average cognitive load during the first sub-period is substantially higher than that of the second sub-period,
wherein the average cognitive load during the first sub-period is at least about the same as the heightened cognitive load.

15. The system of one of the claims 1 - 14, wherein the sensors (610) are configured to track and/or monitor the responses of a person, wherein the responses are at least one of a pupillary response, speeding heart rate, breathing rate, release of certain hormones like adrenalin and cortisol, head movement, eye movement, blink rate, movement of head and/or movements of arms, body posture and movement.

## Patentansprüche

1. System, das Folgendes umfasst:
eine Hörprothesen-Suite; und
eine Dateneingabe-Suite, die so konfiguriert ist, dass sie Daten empfängt, die eine kognitive Belastung eines Empfängers anzeigen, wobei
das System so konfiguriert ist, dass es in einem Hörrehabilitations-Übungsmaschinenmodus arbeitet, in dem das System den Betrieb der Hörprothese automatisch auf der Grundlage von Daten einstellt, die von der Dateneingabe-Suite erhalten wurden, um den Empfänger zu trainieren, wodurch der Empfänger rehabilitiert wird,
die Dateneingabe-Suite eine biometrische Suite enthält; und
das System so konfiguriert ist, dass es den Betrieb der Hörprothese automatisch einstellt, wenn es sich im Übungsmodus befindet, basierend auf biometrischen Daten des Empfängers, die durch die biometrische Suite erhalten wurden, um den Empfänger zu trainieren, wodurch der Empfänger rehabilitiert wird,
wobei die biometrische Suite ein Modul enthält, das so konfiguriert ist, dass es von Sensoren aufgezeichnete biometrische Signale in Echtzeit analysiert und diese Informationen verwendet, um einen mentalen Zustand des Empfängers in Bezug auf seine gegenwärtige kognitive Belastung zu bilden.

2. System nach Anspruch 1, wobei:
die Hörprothesen-Suite so konfiguriert ist, dass sie im Trainingsmodus den Betrieb der Hörprothese automatisch anpasst auf der Grundlage eines Rehabilitationsprogramms, das in Verbindung mit den von der Dateneingabe-Suite erhaltenen Daten arbeitet, um den Empfänger zu trainieren und ihn dadurch zu rehabilitieren.

3. System nach Anspruch 1, wobei:
das System so konfiguriert ist, dass es, wenn es sich im Übungsmodus befindet, den Betrieb der Hörprothese auf der Grundlage der von der Dateneingabe-Suite erhaltenen Daten automatisch anpasst, um die kognitive Belastung des Empfängers zu erhöhen, wenn festgestellt wird, dass die aktuelle kognitive Belastung des Empfängers unter einem vorbestimmten, für die Rehabilitation eingestellten Übungsniveau liegt, oder
das System so konfiguriert ist, dass es, wenn es sich im Übungsmodus befindet, den Betrieb der Hörprothese auf der Grundlage der von der Dateneingabe-Suite erhaltenen Daten und auf der Grundlage historischer Daten automatisch anpasst, um die kognitive Belastung des Empfängers zu variieren, wodurch der Empfänger rehabilitiert wird.

4. System nach Anspruch 1, wobei:
das System so konfiguriert ist, dass es Daten aufzeichnet, die die Einstellung des Betriebs der Hörprothese anzeigen.

5. System nach einem der Ansprüche 1-4, wobei das System konfiguriert ist zum:
Erhalten jeweiliger erster Reaktionen eines Empfängers auf eine Reihe von Geräuschen, denen der Empfänger einer Hörprothese ausgesetzt ist, wobei die ersten Reaktionen in direktem Zusammenhang mit der Fähigkeit des Empfängers stehen, die Reihe von Geräuschen zu hören;
Erhalten jeweiliger zweiter Reaktionen des Empfängers auf die Reihe von Geräuschen, wobei die zweiten Reaktionen von anderer Art sind als die ersten Reaktionen; und
Anpassen einer Hörprothese, die zumindest teilweise auf den ersten Reaktionen und den zweiten Reaktionen basiert.

6. System nach Anspruch 5, wobei:
die zweiten Reaktionen Reaktionen sind, die auf eine kognitive Belastung des Empfängers hinweisen.

7. System nach Anspruch 6, wobei das System weiterhin konfiguriert ist zum:
Bewerten der Hörfähigkeit des Empfängers auf der Grundlage der erhaltenen jeweiligen ersten Reaktionen; Bewertung einer kognitiven Belastung des Empfängers auf der Grundlage der erhaltenen jeweiligen zweiten Reaktionen;
Bestimmen, ob die Bewertung der Hörfähigkeit des Empfängers im Verhältnis zur Bewertung der kognitiven Belastung akzeptabel ist;
Anpassen der Hörprothese zumindest teilweise auf der Grundlage dieser Bestimmung.

8. System nach Anspruch 5, wobei:
die erhaltenen jeweiligen ersten Reaktionen und die erhaltenen jeweiligen zweiten Reaktionen mit der Hörprothese unter Verwendung einer ersten Karte erhalten werden;
das System weiterhin konfiguriert ist zum:
Erhalten jeweiliger dritter Reaktionen des Empfängers auf eine Reihe von Geräuschen, denen der Empfänger der Hörprothese mit der Hörprothese unter Verwendung einer zweiten Karte ausgesetzt ist, wobei die dritten Reaktionen in direktem Zusammenhang mit der Fähigkeit des Empfängers stehen, die Reihe von Geräuschen zu hören;
Erhalten jeweiliger vierter Reaktionen des Empfängers auf die Reihe von Geräuschen, wobei die vierten Reaktionen von anderer Art sind als die dritten Reaktionen;
Vergleichen einer Fähigkeit des Empfängers zu hören, basierend auf den ersten Reaktionen und dritten Reaktionen, und Vergleichen einer kognitiven Belastung des Empfängers, basierend auf den zweiten Reaktionen und den vierten Reaktionen; und
Anpassen der Hörprothese unter Verwendung der ersten Karte auf der Grundlage einer Bestimmung, dass die erste Karte eine geringere kognitive Belastung erfordert als die zweite Karte.

9. System nach Anspruch 5, wobei:
die erhaltenen jeweiligen ersten Reaktionen und die erhaltenen jeweiligen zweiten Reaktionen mit der Hörprothese unter Verwendung einer ersten Karte erhalten werden;
das System ferner konfiguriert ist zum:
Erhalten jeweiliger dritter Reaktionen des Empfängers auf eine Reihe von Geräuschen, denen der Empfänger der Hörprothese mit der Hörprothese unter Verwendung einer zweiten Karte ausgesetzt ist, wobei die dritten Reaktionen in direktem Zusammenhang mit der Fähigkeit des Empfängers stehen, die Reihe von Geräuschen zu hören;
Erhalten von jeweiligen vierten Reaktionen des Empfängers auf die Reihe von Geräuschen, wobei die vierten Reaktionen von anderer Art als die dritten Reaktionen sind;
Vergleichen einer Fähigkeit des Empfängers zu hören, basierend auf den ersten Reaktionen und den dritten Reaktionen, und Vergleichen einer kognitiven Belastung des Empfängers, basierend auf den zweiten Reaktionen und den vierten Reaktionen; und
Anpassen der Hörprothese unter Verwendung der ersten Karte auf der Grundlage einer Bestimmung, dass die erste Karte eine höhere kognitive Belastung erfordert als die zweite Karte.

10. System nach Anspruch 9, wobei das System weiterhin konfiguriert ist zum:
Bestimmen, dass die Fähigkeit des Empfängers, unter Verwendung der ersten Karte zu hören, das Bestimmen einschließt, dass der Empfänger zumindest ungefähr gleich oder besser hören kann als die Fähigkeit des Empfängers, unter Verwendung der zweiten Karte zu hören.

11. System nach Anspruch 1, wobei das System weiterhin konfiguriert ist zum:
Hervorrufen von ersten Hörwahrnehmungen während einer ersten zeitlichen Periode unter Verwendung einer Hörprothese, wobei die Hörprothese auf der Grundlage eines ersten Satzes von Betriebsparametern arbeitet, wenn sie die ersten Hörwahrnehmungen hervorruft;
Empfangen einer Eingabe, die eine durchschnittliche kognitive Belastung des Empfängers anzeigt, die sich aus dem Hervorrufen der ersten Hörwahrnehmungen ergibt; und
Hervorrufen von zweiten Hörwahrnehmungen während einer zweiten zeitlichen Periode unter Verwendung der Hörprothese, wobei die Hörprothese auf der Grundlage eines zweiten Satzes von Betriebsparametern arbeitet, wenn sie die zweiten Hörwahrnehmungen hervorruft, wobei
ein Wechsel von dem ersten Satz von Betriebsparametern zu dem zweiten Satz von Betriebsparametern ausgeführt wird, um die durchschnittliche kognitive Belastung des Empfängers zu erhöhen, die aus dem Hervorrufen der zweiten Hörwahrnehmungen resultiert.

12. System nach Anspruch 11, wobei:
die Hörprothese ein Cochlea-Implantat ist;
der erste Satz von Betriebsparametern eine erste Karte des Cochlea-Implantats ist;
und der zweite Satz von Betriebsparametern eine zweite Karte des Cochlea-Implantats ist.

13. System nach einem der Ansprüche 1 bis 12, wobei:
die kognitive Belastung des Empfängers automatisch unter Verwendung der biometrischen Suite überwacht wird.

14. System nach Anspruch 11, wobei das System ferner konfiguriert ist zum:
Feststellen, dass sich der Empfänger während einer ersten Teilperiode der ersten Zeitperiode in einer ersten Geräuschszene befindet;
Bestimmen, dass sich der Empfänger für eine zweite Teilperiode der ersten Zeitperiode in einer zweiten Geräuschszene befindet, die es wesentlich schwieriger macht, als die erste Geräuschszene zu hören;
Einstellen der Betriebsparameter, so dass die durchschnittliche kognitive Belastung während der ersten Teilperiode und der zweiten Teilperiode zumindest ungefähr gleich ist und zumindest ungefähr gleich der erhöhten kognitiven Belastung ist, oder
Einstellen der Betriebsparameter, so dass die durchschnittliche kognitive Belastung während der ersten Teilperiode wesentlich höher ist als die der zweiten Teilperiode, wobei die durchschnittliche kognitive Belastung während der ersten Teilperiode mindestens ungefähr gleich der erhöhten kognitiven Belastung ist.

15. System nach einem der Ansprüche 1 bis 14, wobei die Sensoren (610) so konfiguriert sind, dass sie die Reaktionen einer Person verfolgen und/oder überwachen, wobei es sich bei den Reaktionen um mindestens eine der folgenden handelt: Pupillenreaktion, Beschleunigung der Herzfrequenz, Atemfrequenz, Freisetzung bestimmter Hormone wie Adrenalin und Cortisol, Kopfbewegung, Augenbewegung, Blinzelrate, Bewegung des Kopfes und/oder Bewegungen der Arme, Körperhaltung und Bewegung.

## Revendications

1. Système, comprenant :
une suite de prothèse auditive ; et
une suite d'entrée de données configurée pour recevoir des données indicatrices d'une charge cognitive d'un receveur, dans lequel
le système est configuré pour fonctionner dans un mode machine d'exercice de réhabilitation de l'audition dans lequel le système ajuste automatiquement le fonctionnement de la prothèse auditive sur la base des données obtenues par la suite d'entrée de données pour exercer le receveur, réhabilitant ainsi le receveur,
la suite d'entrée de données inclut une suite biométrique ; et
le système est configuré pour ajuster automatiquement le fonctionnement de la prothèse auditive lorsqu'elle se trouve dans le mode exercice sur la base des données biométriques du receveur obtenues par la suite biométrique pour exercer le receveur, réhabilitant ainsi le receveur,
dans lequel la suite biométrique inclut un module qui est configuré pour analyser des signaux biométriques enregistrés à partir de capteurs en temps réel et pour utiliser cette information pour former un état mental du receveur par rapport à sa charge cognitive présente.

2. Système selon la revendication 1, dans lequel :
la suite de prothèse auditive est configurée pour, lorsqu'elle se trouve dans le mode exercice, ajuster automatiquement le fonctionnement de la prothèse auditive sur la base d'un programme de réhabilitation qui fonctionne en relation avec les données obtenues par la suite d'entrée de données pour exercer le receveur, réhabilitant ainsi le receveur.

3. Système selon la revendication 1, dans lequel :
le système est configuré pour, lorsqu'il se trouve dans le mode exercice, ajuster automatiquement un fonctionnement de la prothèse auditive sur la base des données obtenues par la suite d'entrée de données pour accroître la charge cognitive sur le receveur sur une détermination que la présente charge cognitive du receveur est inférieure à un niveau d'exercice prédéterminé défini pour une réhabilitation, ou
le système configuré pour, lorsqu'il se trouve dans le mode exercice, ajuster automatiquement un fonctionnement de la prothèse auditive sur la base des données obtenues par la suite d'entrée de données et sur la base de données historiques pour faire varier la charge cognitive sur le receveur réhabilitant ainsi le receveur.

4. Système selon la revendication 1, dans lequel :
le système configuré pour charger des données indicatrices de l'ajustement de fonctionnement de la prothèse auditive.

5. Système selon l'une des revendications 1 à 4, dans lequel le système est configuré pour :
obtenir de premières réactions respectives d'un receveur vers une série de sons soumis au receveur d'une prothèse auditive, les premières réactions étant directement liées à la capacité du receveur d'entendre les séries de sons ;
obtenir de secondes réactions respectives du receveur aux séries de sons, les secondes réactions étant différentes en termes de type des premières réactions ; et
ajuster une prothèse auditive sur la base au moins en partie de à la fois les premières réactions et les secondes réactions.

6. Système selon la revendication 5, dans lequel :
les secondes réactions sont des réactions indicatrices d'une charge cognitive du receveur.

7. Système selon la revendication 6, dans lequel le système est en outre configuré pour :
noter la capacité du receveur à entendre sur la base des premières réactions respectives obtenues ; noter une charge cognitive du receveur sur la base des secondes réactions respectives obtenues ;
déterminer si la note de la capacité du receveur à entendre est acceptable par rapport à la note de la charge cognitive ;
ajuster la prothèse auditive sur la base au moins en partie de la détermination.

8. Système selon la revendication 5, dans lequel :
les premières réactions respectives obtenues et les secondes réactions respectives obtenues sont obtenues avec la prothèse auditive utilisant une première carte ;
le système est en outre configuré pour :
obtenir de troisièmes réactions respectives du receveur à une série de sons soumis au receveur de la prothèse auditive avec la prothèse auditive utilisant une seconde carte, les troisièmes réactions étant directement liées à la capacité du receveur à entendre les séries de sons ;
obtenir de quatrièmes réactions respectives du receveur à des séries de sons, les quatrièmes réactions étant différentes en termes de type des troisièmes réactions ; comparer une capacité du receveur à entendre sur la base des premières réactions et des troisièmes réactions et comparer une charge cognitive du receveur sur la base des secondes réactions et des quatrièmes réactions ; et
ajuster la prothèse auditive en utilisant la première carte sur la base d'une détermination que la première carte requiert une charge cognitive inférieure à celle de la seconde carte.

9. Système selon la revendication 5, dans lequel :
les premières réactions respectives obtenues et les secondes réactions respectives obtenues sont obtenues avec la prothèse auditive utilisant une première carte ;
le système est en outre configuré pour :
obtenir de troisièmes réactions respectives du receveur à une série de sons soumis au receveur de la prothèse auditive avec la prothèse auditive utilisant une seconde carte, les troisièmes réactions étant directement liées à la capacité du receveur d'entendre les séries de sons ;
obtenir de quatrièmes réactions respectives du receveur aux séries de sons, les quatrièmes réactions étant différentes en termes de type des troisièmes réactions ;
comparer une capacité du receveur à entendre sur la base des premières réactions et des troisièmes réactions et comparer une charge cognitive du receveur sur la base des secondes réactions et des quatrièmes réactions ; et
ajuster la prothèse auditive en utilisant la première carte sur la base d'une détermination que la première carte requiert une charge cognitive supérieure à celle de la seconde carte.

10. Système selon la revendication 9, dans lequel le système est en outre configuré pour :
déterminer que la capacité du receveur à entendre en utilisant la première carte inclut une détermination que le receveur peut entendre au moins environ de la même manière ou mieux que la capacité du receveur à entendre en utilisant la seconde carte.

11. Système selon la revendication 1, dans lequel le système est en outre configuré pour :
évoquer de premières perceptions auditives durant une première période temporelle en utilisant une prothèse auditive, dans lequel la prothèse auditive fonctionne sur la base d'un premier ensemble de paramètres opérationnels lors de l'évocation des premières perceptions auditives ;
recevoir une entrée indicatrice d'une charge cognitive moyenne du receveur résultant de l'évocation des premières perceptions auditives ; et
évoquer de secondes perceptions auditives durant une seconde période temporelle en utilisant la prothèse auditive, dans lequel la prothèse auditive fonctionne sur la base d'un second ensemble de paramètres opérationnels lors de l'évocation des secondes perceptions auditives, dans lequel
une commutation depuis le premier ensemble de paramètres opérationnels vers le second ensemble de paramètres opérationnels est exécutée pour accroître la charge cognitive moyenne sur le receveur qui résulte de l'évocation des secondes perceptions auditives.

12. Système selon la revendication 11, dans lequel :
la prothèse auditive est un implant cochléaire ;
le premier ensemble de paramètres opérationnels est une première carte de l'implant cochléaire ;
et le second ensemble de paramètres opérationnels est une seconde carte de l'implant cochléaire.

13. Système selon l'une des revendications 1 à 12, dans lequel :
la charge cognitive du receveur est surveillée automatiquement en utilisant la suite biométrique.

14. Système selon la revendication 11, dans lequel le système est en outre configuré pour :
déterminer que pour une première sous-période de la première période temporelle, le receveur se trouve dans une première scène sonore ;
déterminer que pour une seconde sous-période de la première période temporelle, le receveur se trouve dans une seconde scène sonore qui lui rend sensiblement plus difficile d'entendre que la première scène sonore ;
ajuster les paramètres opérationnels de sorte que la charge cognitive moyenne durant la première sous-période et la seconde sous-période est au moins environ la même et est au moins environ la même que la charge cognitive accrue, ou
ajuster les paramètres opérationnels de sorte que la charge cognitive moyenne durant la première sous-période est sensiblement supérieure à celle de la seconde sous-période, dans lequel la charge cognitive moyenne durant la première sous-période est au moins environ la même que la charge cognitive accrue.

15. Système selon l'une des revendications 1 à 14, dans lequel les capteurs (610) sont configurés pour suivre et/ou surveiller les réponses d'une personne, dans lequel les réponses sont au moins l'une d'une réponse pupillaire, d'une accélération de la fréquence cardiaque, de la fréquence respiratoire, de la libération de certaines hormones telles que l'adrénaline et le cortisol, d'un mouvement de tête, d'un mouvement des yeux, du clignement, du mouvement de la tête et/ou des mouvements des bras, de la posture et du mouvement du corps.
